(19) Europäisches Patentamt
European Patent Office
Office européen des brevets

(11) Veröffentlichungsnummer: **0 149 426 B1**

(12) **EUROPÄISCHE PATENTSCHRIFT**

(45) Veröffentlichungstag der Patentschrift: **04.05.88**

(21) Anmeldenummer: **84810619.1**

(22) Anmeldetag: **14.12.84**

(51) Int. Cl.⁴: **C 07 D249/08**, C 07 D233/60, A 01 N 43/50, A 01 N 43/653, C 07 D405/12

(54) **Mikrobizide.**

(30) Priorität: **20.12.83 CH 6757/83**
**05.06.84 CH 2731/84**

(43) Veröffentlichungstag der Anmeldung: **24.07.85 Patentblatt 85/30**

(45) Bekanntmachung des Hinweises auf die Patenterteilung: **04.05.88 Patentblatt 88/18**

(84) Benannte Vertragsstaaten:
**AT BE CH DE FR IT LI LU NL**

(56) Entgegenhaltungen:
**EP-A- 0 027 177**
**EP-A- 0 055 059**
**EP-A- 0 063 099**
**EP-A- 0 077 479**
**EP-A- 0 118 245**
**EP-A- 2 833 194**
**DE-A- 2 903 653**
**DE-A- 3 126 478**
**US-A- 4 357 340**
**CHEMICAL ABSTRACTS, Vol. 99, No. 17, 24. Oktober 1983, Columbus, Ohio, USA KAMIJO, TETSUHIDE; YAMAMOTO, RYOJI; HARADA, HIROMU; IIZUKA, KINJI "An improved and convenient procedure for the synthesis of 1-substituted imidazoles" Seite 590, Spalte 1, Zusammenfassung-Nr. 139 843q**

(73) Patentinhaber: **CIBA-GEIGY AG**
**Klybeckstrasse 141**
**CH-4002 Basel (CH)**

(72) Erfinder: **Frick, Willy, Dr.**
**Alter Kirchweg 15**
**CH-4148 Pfeffingen (CH)**
Erfinder: **Meyer, Alfred, Dr.**
**St. Galler-Ring 220**
**CH-4054 Basel (CH)**
Erfinder: **Nyfeler, Robert, Dr.**
**Bärenfelserstrasse 8**
**CH-4057 Basel (CH)**

**EP 0 149 426 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue, substituierte 1-Aryl-2-fluor-2-azolyl-alkanone, -ole, -ester und -ether sowie deren Säureadditionssalze und Metallsalzkomplex. Die Erfindung betrifft ferner die Herstellung dieser Substanzen sowie mikrobizide Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von schädlichen Mikroorganismen, vorzugsweise pflanzenschädigenden Pilzen, insbesondere Piricularia-Arten.

Bei den in den erfindungsgemässen Mitteln enthaltenen Wirkstoffen handelt es sich um solche der allgemeinen Formel I

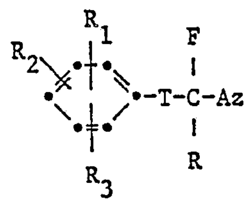

(I)

worin

Az für 1H-1,2,4-Triazol, 4H-1,2,4-Triazol oder 1H-Imidazol steht ;

T für —C(O)—, —CH(OH)— oder für eine der Gruppen

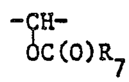 oder 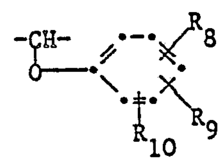 steht, wobei

$R_7$ unsubstituiertes oder durch $C_1$-$C_3$-Alkoxy, Halogen oder Cyano substituiertes $C_1$-$C_6$-Alkyl, $C_3$-$C_6$-Alkenyl, $C_3$-$C_6$-Alkinyl, $C_3$-$C_6$-Cycloalkyl, 2-Furyl, 2-Tetrahydrofuryl oder unsubstituiertes oder durch Halogen, Nitro, $C_1$-$C_3$-Alkyl, $C_1$-$C_3$-Alkoxy, $C_1$-$C_3$-Haloalkyl und/oder $C_1$-$C_3$-Haloalkoxy substituiertes Phenyl oder Benzyl bedeutet ;

$R_8$, $R_9$ und $R_{10}$ unabhängig voneinander für Wasserstoff, Nitro, Halogen, Cyano, $C_1$-$C_3$-Alkyl, $C_1$-$C_3$-Alkoxy, $C_1$-$C_3$-Haloalkoxy, —COO($C_1$-$C_3$-Alkyl), $NH_2$ oder $NHCOCH_3$ stehen ;

R Wasserstoff oder $C_1$-$C_6$-Alkyl bedeutet ;

$R_1$ und $R_2$ unabhängig voneinander für Wasserstoff, Halogen, $C_1$-$C_6$-Alkyl, $C_1$-$C_6$-Alkoxy, $C_1$-$C_6$-Haloalkyl, $C_1$-$C_6$-Haloalkoxy, Nitro und/oder Cyano stehen ; und

$R_3$ Wasserstoff, Halogen, $C_1$-$C_6$-Alkyl, $C_1$-$C_6$-Alkoxy, $C_1$-$C_6$-Haloalkyl, $C_1$-$C_6$-Haloalkoxy, Nitro und/oder Cyano repräsentiert ; unter Einschluss ihrer Säureadditionssalze und Metallsalzkomplexe.

Diejenigen Verbindungen der Formel I, die unter die folgende Formel I' fallen, sind neu :

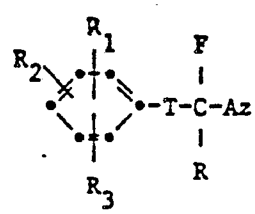

(I')

worin

Az für 1H-1,2,4-Triazol, 4H-1,2,4-Triazol oder 1H-Imidazol steht ;

T für —C(O)—, —CH(OH)— oder für eine der Gruppen

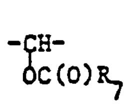 oder 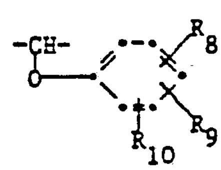 steht, wobei

$R_7$ unsubstituiertes oder durch $C_1$-$C_3$-Alkoxy, Halogen oder Cyano substituiertes $C_1$-$C_6$-Alkyl, $C_3$-$C_6$-Alkenyl, $C_3$-$C_6$-Alkinyl, $C_3$-$C_6$-Cycloalkyl, 2-Furyl, 2-Tetrahydrofuryl oder unsubstituiertes oder durch

Halogen, Nitro, $C_1$-$C_3$-Alkyl, $C_1$-$C_3$-Alkoxy, $C_1$-$C_3$-Haloalkyl und/oder $C_1$-$C_3$-Haloalkoxy substituiertes Phenyl oder Benzyl bedeutet ;

$R_8$, $R_9$ und $R_{10}$ unabhängig voneinander für Wasserstoff, Nitro, Halogen, Cyano, $C_1$-$C_3$-Alkyl, $C_1$-$C_3$-Alkoxy, $C_1$-$C_3$-Haloalkoxy, —COO($C_1$-$C_3$-Alkyl), $NH_2$ oder $NHCOCH_3$ stehen ;

R Wasserstoff oder $C_1$-$C_6$-Alkyl bedeutet ;

$R_1$ und $R_2$ unabhängig voneinander für Wasserstoff, Halogen, $C_1$-$C_6$-Alkyl, $C_1$-$C_6$-Alkoxy, $C_1$-$C_6$-Haloalkyl, $C_1$-$C_6$-Haloalkoxy, Nitro und/oder Cyano stehen ; und

$R_3$ Wasserstoff, Halogen, $C_1$-$C_6$-Alkyl, $C_1$-$C_6$-Alkoxy, $C_1$-$C_6$-Haloalkyl, $C_1$-$C_6$-Haloalkoxy, Nitro und/oder Cyano repräsentiert ;

unter Einschluss ihrer Säureadditionssalze und Metallsalzkomplexe ; mit der Massgabe, dass 1) wenn T gleich —C(O)— ist, R $C_1$-$C_6$-Alkyl darstellt und 2) wenn T gleich C(O)— ist und gleichzeitig von den Substituenten $R_1$, $R_2$ und $R_3$ zwei Reste 2,4-Difluor und der dritte Rest Wasserstoff bedeuten, R $C_2$-$C_6$-Alkyl darstellt.

Wichtige Untergruppen der Verbindungen der Formeln I und I' bilden diejenigen, worin T für —C(O)— oder —CH(OH)— steht, eine weitere, diejenigen, worin T für eine der Gruppen

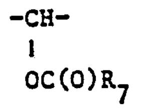 oder 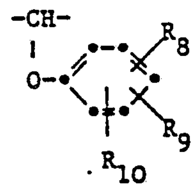 steht und die

übrigen Substituenten wie unter den Formeln I und I' definiert sind.

Unter dem Begriff Alkyl selbst oder als Bestandteil eines anderen Substituenten sind je nach Zahl der angegebenen Kohlenstoffatome beispielsweise folgende Gruppen zu verstehen : Methyl, Ethyl, Propyl, Butyl, Pentyl, Hexyl usw. sowie ihre Isomeren, wie z. B. Isopropryl, Isobutyl, tert.-Butyl, Isopentyl usw. Haloalkyl steht für einen einfach bis perhalogenierten Alkylsubstituenten, wie z.B. $CHCl_2$, $CHF_2$, $CH_2Cl$, $CCl_3$, $CH_2F$, $CH_2CH_2Cl$, CHBr usw. insbesondere $CF_3$. Unter Halogen soll hier und im folgenden Fluor, Chlor, Brom oder Jod, vorzugsweise Fluor, Chlor oder Brom, verstanden werden. Haloalkoxy steht für einen einfach bis perhalogenierten Alkoxyrest, wie z.B. $CF_3O$, $F_2CHO$, $FCH_2O$, $CF_3CF_2O$, $CCl_3CCl_2O$, $CHCl_2CCl_2O$, $CHCl_2CHClO$, $CHBr_2CH_2O$, $CH_2FCH_2O$ usw.

Die vorliegende Erfindung betrifft somit die freien Verbindungen der Formel I, deren Säureadditionssalze und Metallsalzkomplexe. Die freien Verbindungen, insbesondere die 1H-1,2,4-Triazolderivate im Umfang der Formel I sind bevorzugt.

Beispiele addierbarer Säuren sind anorganische Säuren : Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Ameisensäure, Benzolsufonsäure, p-Toluolsulfonsäure, Methansulfonsäure, Maleinsäure, Bersteinsäure, Weinsäure, Fumarsäure, Salizylsäure, Milchsäure oder Sorbinsäure.

Die Metallsalzkomplexe bestehen aus der zugrundeliegenden freien Verbindung der Formel I und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Zirkonium, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d.h. sie können ein oder mehrere organische Molekülanteile als Liganden enthalten. Komplexe mit den Metallen Kupfer, Zink, Mangan, Zinn und Zirkonium sind bevorzugt.

Die Verbindungen der Formel I sind bei Raumtemperatur stabile Oele, Harze oder überwiegend Feststoffe, die sich durch sehr wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen, dabei sind die Triazolylmethylderivate im Umfang der Formel I bevorzugt. Die Wirkstoffe der Formel I zeichnen sich bei niederigen Anwendungskonzentrationen durch eine sehr gute Pflanzen-fungizide Wirkung und problemlose Anwendung aus.

Aufgrund ihrer ausgeprägten mikrobiziden insbesondere jedoch pflanzenfungiziden Wirkung sind folgende Substanzgruppen bevorzugt :

a) Verbindungen der Formel I und der eingeschränkten Formel I', worin Az für 1H-1,2,4-Triazol oder 1H-Imidazol steht ; T für —C(O)— oder —CH(OH)— steht ; R Wasserstoff oder $C_1$-$C_6$-Alkyl bedeutet ; $R_1$ und $R_2$ unabhängig voneinander Wasserstoff, Halogen, $C_1$-$C_3$-Alkyl, $C_1$-$C_3$-Alkoxy, $C_1$-$C_3$-Haloalkyl, $C_1$-

$C_3$-Haloalkoxy, Nitro und/oder Cyano bedeuten; und $R_3$ Wasserstoff, Halogen, $C_1$-$C_3$-Alkyl, $C_1$-$C_3$-Alkoxy, $C_1$-$C_3$-Haloalkyl, $C_1$-$C_3$-Haloalkoxy, Nitro, und/oder Cyano repräsentiert; unter Einschluss ihrer Säureadditionssalze und Metallkomplexe.

b) Verbindungen der Formel I, worin Az für 1H-1,2,4-Triazol oder 1H-Imidazol steht; T für —C(O)— oder —CH(OH)— steht; R Wasserstoff oder $C_1$-$C_6$-Alkyl bedeutet; $R_1$ und $R_2$ unabhängig voneinander Wasserstoff, Fluor, Chlor, Brom, Methyl, Ethyl, Methoxy, Ethoxy, $CF_3$, $C_1$-$C_2$-Haloalkyl, Nitro und/oder Cyano bedeuten; $R_3$ Wasserstoff, Fluor, Chlor, Brom, Methyl, Methoxy, $CF_3$, $OCHF_2$, $OCF_3$, Nitro und/oder Cyano
unter Einschluss ihrer Säureadditionssalze und Metallkomplexe.

c) Verbindungen der Formel I, worin T für —C(O)— steht und die Substituenten R bis $R_3$ wie in Gruppe b definiert sind.

d) Verbindungen der Formel I, worin T für —CH(OH)— steht und die Substituenten R bis $R_3$ wie in Gruppe b definiert sind.

e) Verbindungen der eingeschränkten Formel I', worin Az für 1H-1,2,4-Triazol oder 1H-Imidazol steht; T für eine der Gruppen

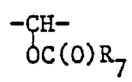 oder 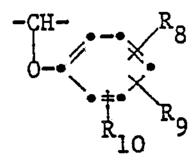 steht, wobei

$R_7$ $C_1$-$C_6$-Alkyl oder durch $C_1$-$C_2$-Alkoxy, Fluor, Chlor oder Brom substituiertes $C_1$-$C_4$-Alkyl, $C_3$-$C_4$-Alkenyl, $C_3$-$C_4$-Alkinyl, Cyclopropyl, Cyclohexyl, 2-Furyl, 2-Tetrahydrofuryl oder unsubstituiertes oder durch Fluor, Chlor, Brom, Methyl, Methoxy, Nitro, $CF_3$, $OCF_2H$ und/oder $OCF_3$ substituiertes Phenyl oder Benzyl bedeutet;

$R_8$, $R_9$ und $R_{10}$ unabhängig voneinander für Wasserstoff, Nitro, Fluor Chlor, Brom, Cyano, Methyl, Methoxy, —COO($CH_3$), $NH_2$ oder $NHCOCH_3$ stehen;

R Wasserstoff oder $C_1$-$C_6$-Alkyl bedeutet;

$R_1$ und $R_2$ unabhängig voneinander für Wasserstoff, Halogen, $C_1$-$C_3$-Alkyl, $C_1$-$C_3$-Alkoxy, $C_1$-$C_3$-Halogalkyl, $C_1$-$C_3$-Haloalkoxy, Nitro und/oder Cyano stehen; und

$R_3$ Wasserstoff, Halogen, $C_1$-$C_6$-Alkyl, $C_1$-$C_3$-Alkoxy, $C_1$-$C_3$-Haloalkyl, $C_1$-$C_3$-Haloalkoxy, Nitro und/oder Cyano repräsentiert;
unter Einschluss ihrer Säureadditionssalze und Metallkomplexe.

f) Verbindungen der Formel I und der eingeschränkten Formel I' worin T für —C(O)— oder —CH(OH)— steht; Az für 1H-1,2,4-Triazol steht; R für Wasserstoff oder $C_1$-$C_4$-Alkyl steht; $R_1$ in ortho-Position und $R_2$ in para-Position stehen und unabhängig voneinander für Fluor, Chlor, Brom, Methyl, Methoxy, $CF_3$, $OCHF_2$, $OCF_3$, Nitro oder Cyano bedeuten; und $R_3$ für Wasserstoff steht; unter Einschluss ihrer Säureadditionssalze und Metallkomplexe.

Besonders bevorzugte Alkanone im Umfang der Formel I sind z.B.:

1-(2, 4-Dichlorphenyl)-2-fluor-2-(1H-1,2,4-triazol-1-yl)-ethanon (Nr. 1.1);
1-(2,4-Dichlorphenyl)-2-fluor-2-(1H-imidazol-1-yl)-ethanon (Nr. 1.2.);
1-(2,4-Dichlorphenyl)-2-fluor-2-(1H-1,2,4-triazol-1-yl)-propanon (Nr. 1.3);
1-(2,4-Dichlorphenyl)-2-fluor-2-(1H-imidazol-1-yl)-propanon (Nr. 1.4);
1-(2,4-Dichlorphenyl)-2-fluor-2-(1H-1,2,4-triazol-1-yl)-butanon (Nr. 1.5);
1-(Phenyl)-2-fluor-2-(1H-1,2,4-triazol-1-yl)-ethanon (Nr. 1.9);
1-(Phenyl)-2-fluor-2-(1H-1,2,4-triazol-1-yl)-propanon (Nr. 1.10);
1-(Phenyl)-2-fluor-2-(1H-1,2,4-triazol-1-yl)-hexanon (Nr. 1.11);
1-(2,4-Dichlorphenyl)-2-fluor-2-(1H-1,2,4-triazol-1-yl)-pentanon (Nr. 1.12);
1-(4-Fluorphenyl)-2-fluor-2-(1H-1,2,4-triazol-1-yl)-hexanon (Nr. 1.15);
1-(4-Chlorphenyl)-2-fluor-2-(1H-1,2,4-triazol-1-yl)-ethanon (Nr. 1.18);
1-(3,4-Dichlorphenyl)-2-fluor-2-(1H-1,2,4-triazol-1-yl)-ethanon (Nr. 1.23);
1-(2,5-Dichlorphenyl)-2-fluor-2-(1H-1,2,4-triazol-1-yl)-ethanon (Nr. 1.25);
1-(2,4-Dimethylphenyl)-2-fluor-2-(1H-1,2,4-triazol-1-yl)-ethanon (Nr. 1.27);
1-(4-Bromphenyl)-2-fluor-2-(1H-1,2,4-triazol-1-yl)-ethanon (Nr. 1.28);
1-(2,4-Difluorphenyl)-2-fluor-2-(1H-1,2,4-triazol-1-yl)-ethanon (Nr. 1.56);
1-(2,3,4-Trichlorphenyl)-2-fluor-2-(1H-1,2,4-triazol-1-yl)-ethanon (Nr. 1.57);
1-(3-Methoxyphenyl)-2-fluor-2-(1H-1,2,4-triazol-1-yl)-ethanon (Nr. 1.60);
1-(2-Methoxyphenyl)-2-fluor-2-(1H-1,2,4-triazol-1-yl)-ethanon (Nr. 1.61);

Unter diesen Alkanonen sind die Verbindungen Nr. 1.1, 1.3 und 1.12 besonders bevorzugt.
Besonders bevorzugte Alkanole im Umfang der Formel I sind z.B.:

1-(2,4-Dichlorphenyl)-2-fluor-2-(1H-1,2,4-triazol-1-yl)-ethanol (Nr. 2.1);

1-(2,4-Dichlorphenyl)-2-fluor-2-(1H-1,2,4-triazol-1-yl)-propanol (Nr. 2.3) ;
1-(2,4-Dichlorphenyl)-2-fluor-2-(1H-imidazol-1-yl)-propanol (Nr. 2.4) ;
1-(2,4-Dichlorphenyl)-2-fluor-2-(1H-1,2,4-triazol-1-yl)-butanol (Nr. 2.5) ;
1-(Phenyl)-2-fluor-2-(1H-1,2,4-triazol-1-yl)-ethanol (Nr. 2.9) ;
1-(Phenyl)-2-fluor-2-(1H-1,2,4-triazol-1-yl)-propanol (Nr. 2.10) ;
1-(Phenyl)-2-fluor-2-(1H-1,2,4-triazol-1-yl)-hexanol (Nr. 2.11) ;
1-(2,4-Dichlorphenyl)-2-fluor-2-(1H-1,2,4-triazol-1-yl)-pentanol (Nr. 2.12) ;
1-(2,4-Dichlorphenyl)-2-fluor-2-(1H-imidazol-1-yl)-butanol (Nr. 2.13) ;
1-(4-Fluorphenyl)-2-fluor-2-(1H-1,2,4-triazol-1-yl)-ethanol (Nr. 2.14) ;
1-(4-Chlorphenyl)-2-fluor-2-(1H-1,2,4-triazol-1-yl)-ethanol (Nr. 2.17) ;
1-(3,4-Dichlorphenyl)-2-fluor-2-(1H-1,2,4-triazol-1-yl)-ethanol (Nr. 2.22) ;
1-(2,5-Dichlorphenyl-2-fluor-2-(1H-1,2,4-triazol-1-yl)-ethanol (Nr. 2.24) ;
1-(2,4-Dimethylphenyl)-2-fluor-2-(1H-1,2,4-triazol-1-yl)-ethanol (Nr. 2.27) ;
1-(4-Bromphenyl)-2-fluor-2-(1H-1,2,4-triazol-1-yl)-ethanol (Nr. 2.28) ;
1-(2,4-Difluorphenyl)-2-fluor-2-(1H-1,2,4-triazol-1-yl)-ethanol (Nr. 2.56) ;
1-(2,3,4-Trichlorphenyl)-2-fluor-2-(1H-1,2,4-triazol-1-yl)-ethanol (Nr. 2.57) ;
1-(3-Methoxyphenyl)-2-fluor-2-(1H-1,2,4-triazol-1-yl)-ethanol (Nr. 2.60) ;
1-(2-Methoxyphenyl)-2-fluor-2-(1H-1,2,4-triazol-1-yl)-ethanol (Nr. 2.61) ;

Bevorzugt sind insbesondere die Alkanole Nr. 2.1 und 2.5.

Besonders bevorzugte Untergruppen von mikrobizid aktiven Ethern und Estern bilden z.B. folgende Verbindungen der Formel I

h) Verbindungen der Formel I, worin Az für 1H-1,2,4-Triazol oder 1H-Imidazol steht ; T für eine der Gruppen

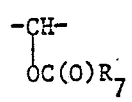 oder 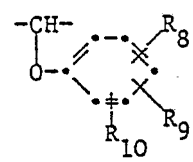 steht, wobei

$R_7$ $C_1$-$C_6$-Alkyl, durch Fluor, Chlor, Brom oder $C_1$-$C_2$-Alkoxy, substituiertes $C_1$-$C_4$-Alkyl, $C_3$-$C_4$-Alkenyl, $C_3$-$C_4$-Alkinyl, Cyclopropyl, Cyclohexyl, 2-Furyl, 2-Tetrahydrofuryl oder unsubstituiertes oder durch Fluor, Chlor, Brom, Nitro, Methyl, Methoxy, $CF_3$, $OCF_3$ und/oder $OCHF_2$ substituiertes Phenyl oder Benzyl steht ;

$R_8$, $R_9$, $R_{10}$ unabhängig voneinander für Wasserstoff, Nitro, Fluor, Chlor, Brom, Methyl, Cyano, Methoxy, —$COOCH_3$, $CF_3$, $NH_2$ oder $NHCOCH_3$ stehen ;

R Wasserstoff oder $C_1$-$C_6$-Alkyl bedeutet ;

$R_1$ und $R_2$ unabhängig voneinander Wasserstoff, Halogen, $C_1$-$C_3$-Alkyl, $C_1$-$C_3$-Alkoxy, $C_1$-$C_3$-Haloalkyl, $C_1$-$C_3$-Haloalkoxy, Nitro und/oder Cyano bedeuten ; und $R_3$ Wasserstoff, Halogen, $C_1$-$C_3$-Alkyl, $C_1$-$C_3$-Alkoxy, $C_1$-$C_3$-Haloalkyl, $C_1$-$C_3$-Haloalkoxy, Nitro und/oder Cyano repräsentiert ;
unter Einschluss ihrer Säureadditionssalze und Metallkomplexe.

i) Verbindungen der Formel I, worin Az für 1H-1,2,4-Triazol oder 1H-Imidazol steht ; T für eine der Gruppen

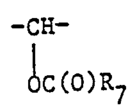 oder 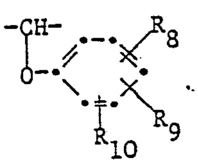 steht, wobei

$R_7$ $C_1$-$C_6$-Alkyl, durch Fluor, Chlor, Brom oder $C_1$-$C_2$-Alkoxy substituiertes $C_1$-$C_4$-Alkyl, $C_3$-$C_4$-Alkenyl, $C_3$-$C_4$-alkinyl, Cyclopropyl, Cyclohexyl, 2-Furyl, 2-Tetrahydrofuryl oder unsubstituiertes oder durch Fluor, Chlor, Brom, Nitro, Methyl, Methoxy, $CF_3$, $OCF_3$ und/oder $OCHF_2$ substituiertes Phenyl oder Benzyl steht ;

$R_8$ $R_9$, $R_{10}$ unabhängig voneinander für Wasserstoff, Nitro, Fluor, Chlor, Brom, Methyl, Cyano, Methoxy, —$COOCH_3$, $CF_3$, $NH_2$ oder $NHCOCH_3$ stehen ;

R Wasserstoff oder $C_1$-$C_6$-Alkyl bedeutet ; $R_1$ und $R_2$ unabhängig voneinander Wasserstoff, Fluor, Chlor, Brom, Methyl, Ethyl, Methoxy, Ethoxy, $CF_3$, $C_1$-$C_2$-Haloalkyl, Nitro und/oder Cyano bedeuten ; $R_3$ Wasserstoff, Fluor, Chlor, Brom, Methyl, Methoxy, $CF_3$, $OCHF_2$, $OCF_3$, Nitro und/oder Cyano repräsentiert,
unter Einschluss ihrer Säureadditionssalze und Metallkomplexe.

k) Verbindungen der Formel I, worin T für die Gruppe

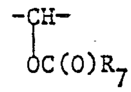 steht, wobei

$R_7$ $C_1$-$C_6$-Alkyl, durch Fluor, Chlor, Brom oder $C_1$-$C_2$-Alkoxy substituiertes $C_1$-$C_4$-Alkyl, $C_3$-$C_4$-Alkenyl, $C_3$-$C_4$-Alkinyl, Cyclopropyl, Cyclohexyl, 2-Furyl, 2-Tetrahydrofuryl oder unsubstituiertes oder durch Fluor, Chlor, Brom, Nitro, Methyl, Methoxy, $CF_3$, $OCF_3$ und/oder $OCHF_2$ substituiertes Phenyl oder Benzyl steht ; und die Substituenten R bis $R_3$ wie in Gruppe b definiert sind.

1) Verbindungen der Formel I, worin T für die Gruppe

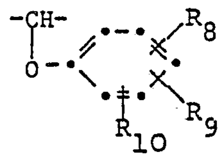 steht, wobei

$R_8$, $R_9$, $R_{10}$ unabhängig voneinander für Wasserstoff, Nitro, Fluor, Chlor, Brom, Methyl, Cyano, Methoxy, —$COOCH_3$, $CF_3$, $NH_2$ oder $NHCOCH_3$ stehen ; und die Substituenten R bis $R_3$ wie in Gruppe b definiert sind.

Innerhalb der Gruppen h bis 1 sind insbesondere jene Verbindungen bevorzugt, worin $R_1$ in der 2-Stellung und $R_2$ in der 4-Stellung des Phenylrings fixiert sind und $R_3$ Wasserstoff bedeutet ; und unter diesen sind besonders diejenigen bevorzugt, worin $R_1$ und $R_2$ für Halogen, insbesondere für Chlor oder Fluor stehen.

n) Verbindungen der Formel I, worin Az für 1H-1,2,4-Triazol steht ; T für eine der Gruppen

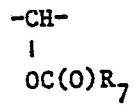 oder 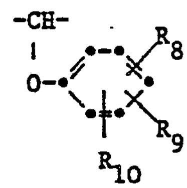 steht ;

wobei $R_7$ für $C_1$-$C_4$-Alkyl oder unsubstituiertes oder durch Fluor, Chlor, Brom, Nitro, Methyl, Methoxy, $CF_3$, $OCF_3$ und/oder $OCHF_2$ substituiertes Phenyl steht ; $R_8$, $R_9$ und $R_{10}$ unabhängig voneinander für Wasserstoff, Fluor, Chlor, Brom, Nitro, Methyl, Methoxy, $CF_3$, $OCF_3$ oder $OCHF_2$ stehen ; R für Wasserstoff oder $C_1$-$C_4$-Alkyl steht ; $R_1$ in der ortho-Position steht ; $R_2$ in der metha-Position steht und beide Reste $R_1$ und $R_2$ unabhängig voneinander für Fluor, Chlor, Brom, Methyl, Methoxy, $CF_3$, $OCHF_2$, $OCF_3$, Nitro oder Cyano stehen und $R_3$ Wasserstoff bedeuten.

Besonders bevorzugte Ether und Ester im Umfang der Formel I sind z.B. :

1-(2,4-Dichlorphenyl)-1-acetoyl-2-fluor-2-(1H-1,2,4-triazol-1-yl)-ethan, (Nr. 5.1),

1-(2,4-Dichlorphenyl)-1-benzoyl-2-fluor-2-(1H-1,2,4-triazol-1-1,2,4-triazol-1-yl)-ethan, (Nr. 5.15),

1-(2,4-Dichlorphenyl)-1-(4-nitrophenoxy)-2-fluor-2-(1H-1,2,4-triazol-1-yl)-ethan (Nr. 6.1).

Es wurde gefunden, dass man die Verbindungen der Formel I sowie deren Säureadditionssalze und Metallkomplexe enthält, indem man ein Keton der Formel II

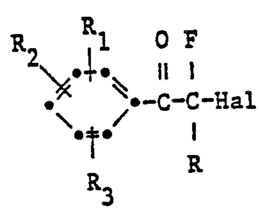 (II)

worin R, $R_1$, $R_2$ und $R_3$ die unter Formel I angegebenen Bedeutungen haben und Hal für Halogen, vorzugsweise Chlor oder Brom steht, mit einem Azol der Formel III

Me—Az (III)

worin Az die unter Formel I angegebenen Bedeutungen hat und Me für Wasserstoff, ein Metallkation, vorzugsweise ein Alkali- oder Erdalkali-Metallkation (z.B. $Na^+$, $K^+$, $Ca^{2+}$ usw.) oder ein Tetraalkylammoniumion, vorzugsweise ein Tetraniederalkylammoniumion wie $N(C_4H_9\text{-}n)^+$, steht, zu einem Alkanon der Formel Ia

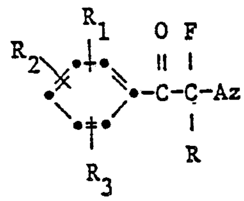

(Ia)

reagieren lässt, worin die Substituenten wie unter Formel I definiert sind und gegebenenfalls das Alkanon der Formel Ia in allgemein üblicher Weise zu einem Alkanol der Formel Ib

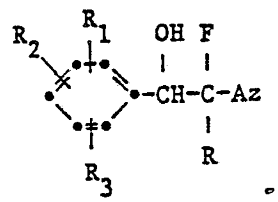

(Ib)

reduziert, wobei die Substituenten wie unter Formel I definiert sind, und gegebenenfalls anschliessend an die Verbindungen der Formel I eine Säure oder ein Metallsalz addiert, oder das Alkanol der Formel Ib durch Reaktion mit einer Verbindung der Formel IV

$$R_7C(O)—Y \qquad (IV)$$

worin Y für Hydroxy, Alkoxy (vorzugsweise $C_1$-$C_6$-Alkoxy), $R_7C(O)O$— oder für Halogen, vorzugsweise Chlor oder Brom steht und $R_7$ die unter Formel I angegebenen Bedeutungen hat, verestert und die Ether der Formel I, dadurch erhält, dass man einen Alkohol der obigen Formel Ib, durch Reaktion mit einer Verbindung der Formel V,

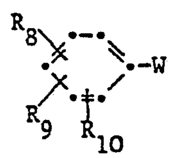

(V)

verethert, und gegebenenfalls eine verfahrensgemäss erhältliche Verbindung durch Reduktion, Umwandlung oder Austausch eines Substituenten in eine andere Verbindung der Formel I überführt, wobei die Substituenten $R_8$, $R_9$ und $R_{10}$ die unter Formel I angegebenen Bedeutungen haben und W für OH oder eine übliche Abgangsgruppe steht, dabei sollen unter einer üblichen Abgangsgruppe hier und im folgenden Substituent wie z. B. Halogene : [wie Fluor, Chlor, Brom oder Job, vorzugsweise Chlor oder Brom] ; Sulfonyloxygruppen, bevorzugt $—OSO_2—R_a$ ; Acyloxygruppen, bevorzugt $—OCO—R_a$ und Isoharnstoffreste, bevorzugt

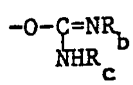

verstanden werden, wobei $R_a$, $R_b$ und $R_c$ unabhängig voneinander für $C_1$-$C_3$-Alkyl, $C_1$-$C_3$-Haloalkyl oder gegebenenfalls durch Halogen, Methyl, Nitro, Trifluormethyl und/oder Methoxy substituiertes Phenyl stehen.

In den Fällen, in denen Phenylether der Formel I hergestellt werden sollen, deren Phenylgruppe in der Etherfunktion in 2- bzw. 4-Position einen elektronenziehenden Rest aufweist, wie z. B. $—NO_2$, CN, COOAlkyl etc., kann man diesen Rest z. B. durch Reduktion ($NO_2 \longrightarrow NH_2$) oder Umwandlung bzw. Austausch (z. B. gegen Halogen) leicht in einen anderen Substituenten ($R_8$ bis $R_{10}$) überführen und gelangt so zu in 2- bzw. 4-Stellung substituierten Verbindungen der Formel I, die sonst in nur ungenügenden Ausbeuten zugänglich sind.

Das Verfahren wird in Ab- oder vorzugsweise Anwesenheit von reaktionsinerten Lösungsmitteln durchgeführt. So wird die Reaktion von II mit III bevorzugt in einem relativ polaren, jedoch reaktionsinerten Lösungsmittel durchgeführt, z. B. N,N-Dimethyl-formamid, N,N-Dimethylacetamid, Dimethylsulfoxid, in Nitrilen wie Acetonitril, Benzonitril, Propionitril usw., in Ketonen wie Aceton, Methylethylketon usw., in

etherartigen Lösungsmitteln wie Tetrahydrofuran oder Dioxan und anderen mehr. Derartige Lösungsmittel können in Kombination mit anderen üblichen reaktionsinerten Lösungsmitteln, wie aliphatischen oder aromatischen Kohlenwasserstoffen, z. B. Benzol, Toluol, Xylol, Hexan, Petrolether, Chlorbenzol, Nitrobenzol u. a. verwendet werden. Die Reaktion wird bei Temperaturen zwischen 0° und 150 °C, vorzugsweise 20° bis 100 °C und erfolgt bei Normaldruck, gegebenenfalls bei erhöhtem Druck.

Die Reaktion wird vorteilhafterweise in Gegenwart von Kondensationsmitteln oder von säurebindenden Mitteln durchgeführt. Als solche kommen organische und anorganische Basen in Betracht, z. B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, KOH, NaH, $Ca(OH)_2$, $KHCO_3$, $Ca(HCO_3)_2$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, $C_3H_7$-nONa usw. In einigen Fällen kann es von Vorteil sein, wenn man das freie Azol III (M = Wasserstoff) zuerst, z. B. in situ mit einem Alkoholat, in das entsprechende Salz überführt und dann in An- und Abwesenheit einer der genannten Basen mit dem Keton der Formel II umsetzt. Bei der Herstellung der 1,2,4-Triazolylderivate entstehen im allgemeinen parallel auch 1,3,4-Triazolylisomere, die sich auf übliche Weise, z. B. mit unterschiedlichen Lösungsmitteln, voneinander trennen lassen. Entstehender Halogenwasserstoff kann in manchen Fällen auch mittels Durchleiten von Inertgas, z. B. Stickstoff, aus dem Reaktionsgemisch vertrieben oder durch Zugabe von Molekularsieb absorbiert werden.

Die erfindungsgemässe Reduktion (Ia zu Ib) erfolgt in an sich bekannter Weise, z. B. durch Reaktion mit Metallhydriden wie $LiAlH_4$, $NaBH_4$, $BH_3$ usw. (vgl. H.O. House « Modern Synthetic Reactions » 1972, p. 45 ff und p. 154). Arbeitet man mit Metallhydriden, so kommen als Verdünnungsmittel für die erfindungsgemässe Reduktion polare organische Lösungsmittel in Frage. Dies sind bei $NaBH_4$ z. B. Niederalkanole, wie Methanol, Ethanol, Butanol, Isopropanol ansonsten Ether, wie Dialkylether, so z. B. Diethylether oder Tetrahydrofuran. Die Reduktion wird im allgemeinen bei 0 bis 30 °C, vorzugsweise bei 0 bis 20 °C durchgeführt. Hierzu setzt man auf 1 Mol des Ketons der Formel (Ib) etwa 1 Reaktionsäquivalent eines Metallhydrids, wie Natriumborhydrid oder Lithiumaluminiumhydrid, ein. Zur Isolierung der reduzierten Verbindungen der Formel Ib wird der Rückstand in verdünnter Salzsäure aufgenommen, anschliessend alkalisch eingestellt und mit einem organischen Lösungsmittel extrahiert. Die weitere Aufarbeitung erfolgt nach allgemein üblichen Methoden.

Reduziert man mit Aluminiumisopropylat, so kommen als Verdünnungsmittel für die erfindungsgemässe Reduktion bevorzugt Alkanole, wie Isopropanol, oder übliche inerte Kohlenwasserstoffe, wie Benzol oder Toluol in Frage. Die Reaktionstemperaturen können auch in diesem Fall in einem grösseren Bereich variieren ; im allgemeinen arbeitet man zwischen 20 und 120 °C, vorzugsweise bei 50 bis 100 °C. Zur Durchführung der Reduktion setzt man auf 1 Mol des Ketons der Formel Ia etwa I bis 2 Mol Aluminiumisopropylat ein. Zur Isolierung der reduzierten Verbindungen der Formel Ib wird überschüssiges Lösungsmittel durch Destillation im Vakuum entfernt und die entstandene Aluminiumverbindung mit verdünnter Schwefelsäure oder Natronlauge zersetzt. Die weitere Aufarbeitung erfolgt nach allgemein üblichen Methoden.

Die Azole der Formel III sind allgemein bekannt.

Die α-Halogen-α-Fluorketone II sind z. T. bekannt [vgl. F. Bergmann et al., J. Am. Chem. Soc. 79, 4178 (1957)] oder lassen sich nach an sich bekannten Methoden herstellen, so z. B. durch übliche Halogenierung der zugrundeliegenden α-Fluorketone der Formel IV

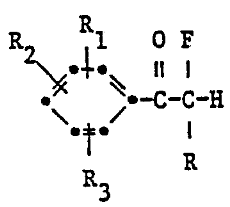

(IV)

worin die Substituenten wie unter Formel I definiert sind. Die α-Fluorketone sind z. T. bekannt [Houben-Weil, Band 5/3, Seiten 211, 246 und 494, sowie F. Bergmann et al. J. Am. Chem. Soc. 76, 4137 (1954)] oder sind überdies analog zu den bekannten α-Fluor-Ketonen aus den entsprechenden bekannten α-Brom-Ketonen durch üblichen Austausch des Broms gegen Fluor zugänglich oder können auch durch Acylierung des zugrundeliegenden Aromaten mit fluorierten Carbonsäurederivaten z. B. nach Friedel-Crafts hergestellt werden.

Säureadditions-Salze der Formel I können in einfacher Weise nach üblichen Salzbildungsmethoden, z. B. durch Lösen einer Verbindung der Formel I in einem geeigneten inerten Lösungsmittel und Hinzufügen der entsprechenden Säure, z. B. einer Halogenwasserstoffsäure wie HC1, erhalten werden und in bekannter Weise, z. B. durch Abfiltrieren isoliert und gegebenenfalls durch Waschen mit einem inerten organischen Lösungsmittel gereinigt werden.

Zur Herstellung von Metallsalz-Komplexen der Formel I kommen vorzugsweise Salze von denjenigen

Anionen und Kationen in Betracht, die bereits im Zusammenhang mit der Beschreibung der erfindungsgemässen Metallsalz-Komplexe als bevorzugt genannt wurden.

Die Metallsalz-Komplexe der Formel I können in einfacher Weise nach üblichen Verfahren erhalten werden, so z. B. durch Lösung des Metallsalzes in einem Alkohol, z. B. Ethanol und Hinzufügen zur freien Verbindung der Formel I. Man kann Metallsalz-Komplexe in bekannter Weise, z. B. durch abfiltrieren, isolieren und beispielsweise durch Umkristallisation reinigen.

Bei den beschriebenen Veretherungs- und Veresterungsreaktionen können reaktionsinerte Lösungs- oder Verdünnungsmittel eingesetzt werden. In Frage kommen beispielsweise aliphatische und aromatische Kohlenwassertoffe wie Benzol, Toluol, Xylole, Petrolether ; halogenierte Kohlenwasserstoff wie Chlorbenzol, Methylenchlorid, Ethylenchlorid, Chloroform, Tetrachlorkohlenstoff, Tetrachlorethylen ; Ether und etherartige Verbindungen wie Dialkylether (Diethylether, Diisopropylether, tert.-Butylmethylether usw.), Anisol, Dioxan, Tetrahydrofuran ; Nitrile wie Acetonitril, Propionitril ; N,N-dialkylierte Amide wie Dimethylformamid ; Dimethylsulfoxid ; Ketone wie Aceton, Diethylketon, und Gemische solcher Lösungsmittel untereinander. In manchen Fällen kann das Acylierungs- oder Veretherungsmittel selbst als Lösungsmittel eingesetzt werden.

Bevorzugte Lösungsmittel sind Ether, Aceton, Chloroform, Dioxan, Dimethylformamid und Dimethylsulfoxid.

Bei der Veresterung von Ib und IV zu I kann die Gegenwart eines Reaktionskatalysators wie beispielsweise Dimethylformamid von Vorteil sein.

Die Reaktionstemperaturen liegen beim Verethern und Verestern zwischen 0° und 180 °C, vorzugsweise 10° und 80 °C bzw. am Siedepunkt des Lösungsmittels bzw. Lösungsmittelgemisches. In manchen Fällen ist die Verwendung von säurebindenden Mitteln bzw. Kondensationsmitteln vorteilhaft. Als solche kommen organische und anorganische Basen in Betracht, z. B. tertiäre Amine wie Trialkylamine (Trimethylamin, Triethylamin, Tripropylamin usw.), Pyridin und Pyridinbasen (4-Dimethylaminopyridin, 4-Pyrrolidiylaminopyridin usw.), Oxide und Hydroxide, Carbonate und Hydrogencarbonate von Alkali- und Erdalkalimetallen sowie Alkaliacetate (KOH, NaOH, $Na_2CO_3$, $NaHCO_3$, CaO, $Ca(OH)_2$, $Ba(OH)_2$, $NaOC(O)CH_3$).

Entstehender Halogenwasserstoff kann in manchen Fällen auch mittels Durchleiten von Inertgas, z. B. Stickstoff, aus dem Reaktionsgemisch vertrieben werden oder durch Zusatz von Molekularsieb aus dem Gemisch entfernt werden.

Die Weiterreaktion von Ib mit V erfolgt in den Fällen, in denen W in Formel V für eine übliche Abgangsgruppe steht in Abwesenheit oder bevorzugt in Anwesenheit eines der obigen reaktionsinerten Lösungsmittels. Auch Gemische solcher Lösungsmittel untereinander oder mit anderen üblichen inerten organischen Lösungsmitteln, z. B. mit aromatischen Kohlenwasserstoffen wie Benzol, Toluol, Xylolen usw. können verwendet werden. Es kann aber auch von Vorteil sein, dass man den Alkohol der Formel Ib zuerst auf an sich bekannte Weise, z. B. durch Reaktion mit einer starken Base, in ein geeignetes Metallsalz überführt.

Geeignete starke Basen sind z. B. Alkali- und Erdalkalihydride (NaH, KH, $CaH_2$ usw.) und Alkaliorganische Verbindungen wie z. B. Butyllithium oder Alkali-tert.-Butoxid, darüberhinaus können auch Alkalihydroxide, wie NaOH oder KOH eingesetzt werden, wenn man in einem wässrigen Zweiphasensystem und in Anwesenheit eines Phasentransferkatalysators arbeitet.

Man kann jedoch auch den Alkohol der Formel Ib, vor der Weiterreaktion zuerst auf übliche Weise in ein Alkalialkoholat überführen und dann mit einer Verbindung der Formel V (worin W für eine Abgangsgruppe steht) umsetzen, dabei arbeitet man vorteilhalterweise in Gegenwart eines Kronenethers. Bei M = K, insbesondere in Gegenwart von 18 Krone-6 ; bei M = Na, insbesondere in Gegenwart von 15 Krone-5. Dabei wird die Reaktion zweckmässigerweise in einem reaktionsinerten Medium durchgeführt. Als Lösungsmittel eignen sich z. B. Ether und etherartige Verbindungen z. B. Diniederalkylether (Diethylether, Diisopropylether, tert.-Butylmethylether usw.), Tetrahydrofuran, Dioxan und aromatische Kohlenwasserstoffe wie Benzol, Toluol oder Xylole.

Für die organische, mit Wasser nicht mischbare Phase kommen dabei z. B. folgende Lösungsmittel in Frage : Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw. Beispiele geeigneter Phasentransfer-Katalysatoren sind : Tetraalkylammoniumhalogenide, -hydrogensulfate oder -hydroxide wie Tetrabutylammoniumchlorid, -bromid, -jodid ; Triethylbenzylammoniumchlorid, -bromid ; Tetrapropylammoniumchlorid, -bromid, -jodid ; usw. Als Phasentransfer-Katalysatoren kommen auch Phosphonium-Salze in Betracht. Die Reaktionstemperaturen liegen im allgemeinen zwischen 30° und 130 °C, bzw. am Siedepunkt des Lösungsmittels oder Lösungsmittelgemisches.

In den Fällen, in denen W in Formel V für Hydroxygruppen stehen, wird vorteilhafterweise eine Kondensationsreaktion durchgeführt. Beide Reaktanden werden in einem geeigneten Lösungenmittel unter Rückfluss erhitzt.

Hierbei können grundsätzlich Lösungsmittel eingesetzt werden, die sich gegenüber den Reaktionspartnern inert verhalten und zweckmässigerweise mit Wasser Azeotrope bilden. Es eignen sich hierzu beispielsweise aromatische Kohlenwasserstoffe, wie Benzol, Toluol, Xylole oder halogenierte Kohlenwasserstoffe, wie Dichlormethan, Chloroform, Tetrachlorkohlenstoff, 1,2-Dichloräthan, Tetrachloräthylen, Chlorbenzol, aber auch ätherartige Verbindungen, wie tert.-Butylmethyläther, Dioxan und andere. In manchen Fällen kann die Verbindung der Formel III selbst als Lösungsmittel verwendet werden. Bei dieser Kondensationsreaktion arbeitet man zweckmässigerweise in Gegenwart einer starken Säure, z. B. Paratoluolsulfonsäure und bei Siedetemperaturen der azeotropen Mischung.

Zur Herstellung der Ether der Formel I kann man auch die freie OH-Gruppe in den Verbindungen der Formel Ib, zuerst gegen eine der obengenannten, üblichen Abgangsgruppen W austauschen und anschliessend mit einer Verbindung der Formel V (mit W = OH) umsetzen.

Der Austausch der freien Hydroxylgruppe in den Verbindungen der Formel Ib gegen eine Abgangsgruppe W wird bevorzugt in einem reaktionsinerten Lösungsmittel durchgeführt. Beispiele solcher Lösungsmittel sind : Aromatische und aliphatische Kohlenwasserstoffe wie Benzol, Toluol, Xylole, Petrolether, Ligroin oder Cyclohexan ; halogenierte Kohlenwasserstoffe wie Chlorbenzol, Methylenchlorid, Ethylenchlorid, Chloroform, Tetrachlorkohlenstoff oder Tetrachloretylen ; Ether und etherartige Verbindungen wie Diethylether, Diisopropylether, t-Butylmethylether, Dimethoxyethan, Dioxan, Tetrahydrofuran oder Anisol ; Ester wie Ethylacetat, Propylacetat oder Butylacetat ; Nitrile wie Acetonitril oder Verbindungen wie Dimethylsulfoxid, Dimethylformamid und Gemische solcher Lösungsmittel untereinander.

Das beschriebene Herstellungsverfahren ist einschliesslich aller Varianten ein Bestandteil der vorliegenden Erfindung.

Die Ausgangsprodukte der Formeln IV und V sind allgemein bekannt oder lassen sich nach an sich bekannten Methoden herstellen.

Die Verbindungen der Formel I

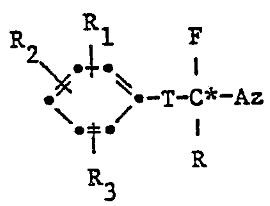

(I)

besitzen stets in Nachbarstellung zur Funktion T ein asymmetrisches Kohlenstoffatom *C und können daher in zwei enantiomeren Formen vorliegen. Im allgemeinen entsteht bei der Herstellung dieser Substanzen ein Gemisch beider Enantiomeren, dieses lässt sich auf übliche Weise, z. B. durch fraktionierte Kristallisation von Salzen mit optisch aktiven, starken Säuren, in die reinen optischen Antipoden aufspalten. Die Enantionmeren können unterschiedliche biologische Wirkungen aufweisen, so kann z. B. bei der einen Form eine blattfungizide und bei der anderen eine bodenfungizide Wirkung im Vordergrund stehen. Auch kann bei gleichem Wirkungsspektrum ein gradueller Aktivitätsunterschied auftreten. Im Falle von T = —*CH(OH)— tritt ein weiteres Asymmetrie-Zentrum (*) auf, was zur Existenz von diastereomeren Gemischen führt (threo- und erythro-Formen), die mittels physikalischer Methoden getrennt werden können. Die einzelnen Isomeren unterscheiden sich wie folgt :

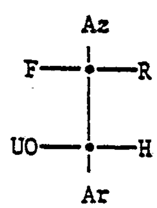 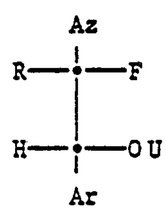 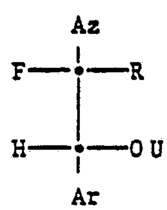 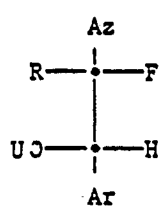

wobei Ar für die Gruppe

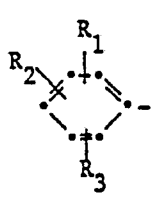 und U für OH, —C(O)$R_7$ oder 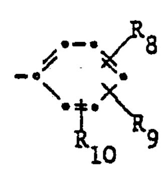

stehen und die Substituenten im übrigen wie unter Formel I definiert sind.

Die vorliegende Erfindung betrifft alle reinen Enantiomeren bzw. Diastereomeren und deren Gemische untereinander.

Das beschriebene Herstellungsverfahren ist, einschliesslich aller Teilschritte, ein wichtiger Bestandteil der vorliegenden Erfindung.

Triazolyl-alkanone und -alkanole der allgemeinen Formel X

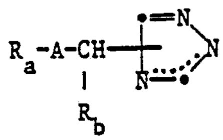

(X)

mit

$R_a$ = Alkyl, Cycloalkyl, gegebenenfalls substituiertes Aryl ;

$R_b$ = H, Alkyl, Cycloalkyl, gegebenenfalls substituiertes Aryl ;

$R_a + R_b$ = aliphatischer Ring.

A = —C(O)—, —CH(OH)—

werden in der Deutschen Offenlegungsschrift DE-2, 431, 407 als Fungizide, insbesondere gegen Pilze der Spezies Erysiphe, Podosphaera, Venturia und Fusicladium beschrieben.

Die Europäische Patentanmeldung A-O 077 479 offenbart mit einem Phenoxyrest substituierte Phenylazolylmethyl-ketone und -carbinole, für welche eine fungizide Aktivität angegeben wird. Ferner sind aus der Europäischen Patentanmeldung A-O 118 245 fluorierte Triazolacetophenon-Derivate als Zwischenprodukte zur Herstellung fungizider Bis-triazolylphenyl-alkanole bekannt.

Es wurde nun überraschend gefunden, dass die Einführung eines Fluoratoms in die zur Azolgruppe nachbarständige Methylengruppe zu einer signifikaten Verschiebung des Wirkungsspektrums führt. Im Gegensatz zu den Verbindungen aus der DE-2, 431, 407 zeigen die erfindungsgemässen, fluorierten Substanzen der Formel I nämlich eine zusätliche, ausgeprägte Aktivität gegen Piricularia-Arten, insbesondere Piricularia oryzae (rice blast). Der Erreger, der Pilz Piricularia oryzae gehört zu den Fungi imperfecti und bildet als Fruktifikationsorgane nur Konidiosporen, eine asexuelle Nebenfruchtform, aus. Er stellt einer der wirtschaftlich wichtigsten Schadfaktoren in Reiskulturen dar, da er die Reispflanzen in allen Wuchsstadien (Sämlinge, Blätter, Stengel, Aehren usw.) und während der ganzen Wachstumsperiode befällt. Je nach dem Angriffsort des Pilzes und dem Stadium, in dem sich die Reispflanzen bei der Infektion befinden, werden verschiedene englische Bezeichnungen für die Krankheit verwendet : z. B. « Leaf blast » für Blattflecken, die zum Absterben der Blätter und damit zu einem gestörten Wachstum führen können ; « Ear blast » für Infektionen an der Rispe oder Teilen der Rispe ; « Neckblast » für den Befall der Rispenbasis, wodurch die Rispe abknickt und keine oder nur anormale Körner gebildet werden ; « Node blast » für das Abknicken der Halme an den Nodien. Piricularia oryzae verbreitet sich durch Konidosporen, die bei feuchter Witterung in Massen gebildet werden und vom Wind über weite Strecken transportiert werden. In Gebieten mit längerer Unterbrechung der Reiskultur im Laufe des Jahres werden zu Beginn der Anbausaison Konidiosporen auf Sämlingen aus infizierten Samen, auf Ernterückständen, auf dem Feld gelagerten Stroh und befallenen Gräsern gebildet. Wenn das ganze Jahr hindurch Reis angebaut wird, stehen häufig Kulturen verschiedenen Alters nebeneinander, so dass hier die Uebertragung der Krankheit durch die sich ständig bildenden Konidien leicht vor sich gehen kann. Der Piriculariabefall führt im allgemeinen zu sehr hohen Ernteausfällen und stellt in Anbetracht der Tatsache, dass Reis nach Weizen und Mais unter den getreideartigen Feldkulturen derzeit die drittgrösste Anbaufläche beansprucht, zu Schäden von grosser wirtschaftlicher Tragweite.

Mit den Wirkstoffen der Formel I kann der Piriculariabefall in allen Wuchsstadien erfolgreich und einfach bekämpft werden. Darüberhinaus sind die Wirkstoffe der Formel I auch gegen die den folgenden Klassen angehörenden phytopathogenen Pilze wirksam : Fungi imperfecti (z. B. Botrytis, Helminthosporium, Fusarium Septoria, Cercospora und Alternaria) ; Basidiomyceten (z. B. die Gattungen Hemileia, Rhizocotonia, Puccinia) ; Ascomyceten (z. B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Ferner lassen sich Verbindungen der Formel I erfolgreich zum Schutz verderblicher Waren pflanzlicher oder tierischer Herkunft einsetzen. Sie bekämpfen z. B. Schimmelpilze wie Penicillium, Aspergillus, Rhizopus, Fusarium, Helminthosporium, Nigrospora und Alternaria sowie Bakterien wie Buttersäurebakterien und Hefen wie Candida.

Die Verbindungen der Formel I können somit im Vorratsschutz (z. B. Getreide, Silagen, Heu usw.) eingesetzt werden.

Die Verbindungen der Formel I zeigen somit ein für praktische Bedürfnisse sehr günstiges Mikrobizid-Spektrum gegen phytopathogene Pilze und Bakterien. Sie besitzen insbesondere sehr vorteilhafte präventive und zusätzlich systemische Eigenschaften und lassen sich zum Schutz von zahlreichen Kulturpflanzen, vorzugsweise jedoch in Reiskulturen einsetzen. Mit den Wirkstoffen der Formel I können an Pflanzen oder an Pflanzenteilen (Früchte, Blüten, Laubwerk, Stengel, Knollen Wurzeln) von unterschiedlichen Nutzkulturen die auftretenden Mikroorganismen eingedämmt oder

vernichtet werden, wobei auch später zuwachsende Pflanzenteile von derartigen Mikroorganismen verschont bleiben.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. vor allem zur präventiven und systemischen Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Neben der Hauptkultur Reis, gelten als Zielkulturen für die hierin offenbarten Indikationsgebiete im Rahmen dieser Erfindung beispielsweise folgende Pflanzenarten : Getreide : (Weizen, Gerste, Roggen, Hafer, Sorghum und Verwandte) ; Rüben : (Zucker- und Futterrüben) ; Kern-, Stein- und Beerenobst : (Aepfel, Birnen, Pflaumen, Pfirsiche, Mandeln, Kirschen, Erd-, Him- und Brombeeren) ; Hülsen-früchte : (Bohnen, Linsen, Erbsen, Soja) ; Oelkulturen : (Raps, Senf, Mohn, Oliven, Sonnenblumen, Kokos, Rizinus, Kakao, Erdnüsse) ; Gurkengewächse ; (Kürbis, Gurken, Melonen) ; Fasergewächse : (Baumwolle, Flachs, Hanf, Jute) ; Citrusfürchte : (Orangen, Zitronen, Pampelmusen, Mandarinen) ; Gemüsesorten : (Spinat, Kopfsalat, Spargel, Kohlarten, Möhren, Zwiebeln, Tomaten, Kartoffeln, Paprika) ; Lorbeergewächse : (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z. B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z. B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha ; bevorzugt 100 g bis 2 kg AS/ha, insbesondere bei 200 g bis 600 g AS/ha.

Die Formulierungen d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reducktion der Aufwandmenge führen können, sind ferner natürliche (tierische oder pflanzliche) oder synthetische Phospholipide aus der Reihe der Kephaline und Lecithine, wie z. B. Phosphatidyl-ethanolamin, Phosphatidylserin, Phosphatidylcholin, Sphingomyelin, Phosphatidylinosit, Phosphatidylglycerin, Lysolecithin, Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z. B. Phosphatidylocholin-Mischungen. Synthetische Phospholipide sind z. B. Dioctanoylphosphatidylcholin und Dipalmitoylphosphatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder- sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürliche Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einem Fettsäurerest mit 8-22 C-Atomen. Alkylarylsulfonate sind z. B. die Na-, Ca- oder Triethanolaminsalze der Dodecylbenzolsulfonsäure, der Dibutylnaphthalinsulfonsäure, oder eines Naphthalinsulfonsäure-Form- aldehydkondensationsproduktes.

Ferner kommen auch entsprechende Phosphate wie z. B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 g Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z. B. das Stearyltrimethylammoniumchlorid oder das Benzyl-bis (2-chlorethyl) ethylammoniumbromid.

Die in der Formulierungstechnik gebräuchlichen Tenside sind u. a. in folgenden Publikationen beschrieben :

« Mc Cutcheon's Detergents and Emulsifiers Annual » BC Publishing Corp., Ridgewood New Jersey, 1981.

Helmut Stache « Tensid-Taschenbuch » Carl Hanser-Verlag München/Wien 1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Druckangaben erfolgen in Millibar mb, oder Bar b.

## Herstellungsbeispiele

### Beispiel H1

Herstellung von

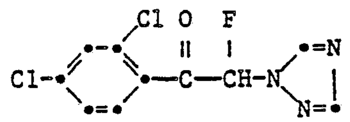

1-(2,4-Dichlorphenyl)-2-fluor-2-(1H-1,2,4-triazol-1-yl)ethanon

a) Herstellung der Vorstufe :

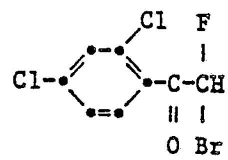

1-(2,4-Dichlorphenyl)-2-brom-2-fluorethanon

Zu einer Lösung von 20,7 g α-Fluor-2,4-dichloracetophenon in 100 ml Tetrachlorkohlenstoff wurde bei 40° bis 45 °C eine Lösung von 16 g Brom in 100 ml Tetrachlorkohlenstoff zugegeben. Nach ca. 1 Stunde hatte sich die braune Lösung entfärbt. Es wurde noch 1 Stunde weitergerührt und anschliessend mit wässriger Natriumhydrogencarbonatlösung ausgeschüttelt und im Vakuum eingedampft. Der ölige Rückstand wurde dann im Hochvakuum destilliert. Ausbeute 17 g. Sdp. 89-92 °C/0,02 mbar.

b) Herstellung des Endproduktes :

2,9 g 1,2,4-Triazol und 4 ml Diisopropylethylamin werden in 30 ml Acetonitril gegeben. Nach schwach exothermer Reaktion entsteht eine hellbraune Lösung. Zu dieser Lösung lässt man bei 5-10 °C unter Rühren 5,7 g 1-(2,4-Dichlorphenyl)-2-brom-2-fluorethanon zutropfen und hält das Reaktionsgemisch 1 Stunde bei 5-10 °C. Anschliessend wird noch 4 Stunden bei Raumtemperatur weitergerührt. Danach wird das Reaktionsgemisch eingeengt und der Rückstand zwischen Eiswasser und Diethylether verteilt. Die etherische Phase wird abgetrennt, über Natriumsulfat getrocknet, filtriert und eingeengt. Der Rückstand wird säulenchromatographisch über Kieselgel gereinigt. Ausbeute 2,4 g. Das aus Diisopropylether umkristallisierte Produkt schmilzt bei 66-67 °C.

### Beispiel H2

Herstellung von

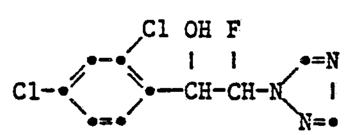

1-(2, 4-Dichlorphenyl)-2-fluor-2-(1H-1, 2, 4-triazol-1-yl)ethanol

Zu einer Suspension von 2,4 g 1-(2,4-Dichlorphenyl)-2-fluor-2-(1H-1,2,4-triazol-1-yl)ethanon in 9,5 ml Methanol und 0,5 ml Wasser werden unter Rühren bei 5-10 °C 0,12 g Natriumborhydrid eingetragen und die resultierende Mischung zuerst 1 Stunde bei 0-5 °C und dann 2 Stunden bei Raumtemperatur gerührt, wobei allmählich eine klare Lösung entsteht. Diese Lösung wird dann auf Eiswasser gegossen und das Gemisch zweimal mit Dichlormethan extrahiert. Die vereinigten Extrakte werden über Natriumsulfat getrocknet, filtriert und eingeengt. Nach Hochvakummdestillation des Rückstands erhält man den obigen Alkohol als Diastereomerengemisch. Sdp. des Rohprodukts 150-160 °C/0,07 mbar. Nach Kristallisation aus Diethylether/Diisopropylether erhält man das gereinigte Diastereomerengemisch in Form beiger

Kristalle. Schmelzintervall 103-112 °C.

Beispiel H3

Herstellung von

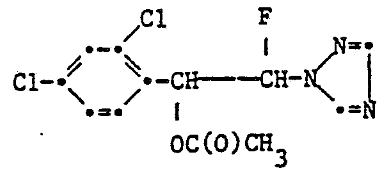

(1.1)

1(-2,4-Dichlorphenyl)-1-acetoyl-2-fluor-2-(1H-1,2,4-triazol-1-yl)-ethan

a) Herstellung des Ausgangsproduktes

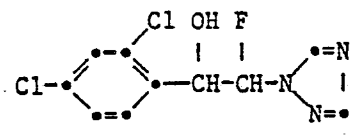

1-(2,4-Dichlorphenyl)-2-fluor-2-(1H-1,2,4-triazol-1-yl)ethanol

Zu einer Suspension von 2,4 g 1-(2,4-Dichlorphenyl)-2-fluor-2-(1H-1,2,4-triazol-1-yl)ethanon in 9,5 ml Methanol und 0,5 ml Wasser werden unter Rühren bei 5-10 °C 0,12 g Natriumborhydrid eingetragen und die resultierende Mischung zuerst 1 Stunde bei 0-5 °C und dann 2 Stunden bei Raumtemperatur gerührt, wobei allmählich eine klare Lösung entsteht. Diese Lösung wird dann auf Eiswasser gegossen und das Gemisch zweimal mit Dichlormethan extrahiert. Die vereinigten Extrakte werden über Natriumsulfat getrocknet, filtriert und eingeengt. Nach Hochvakuumdestillation des Rückstands erhält man den obigen Alkohol als Diastereomerengemisch. Sdp. des Rohprodukts 150-160 °C/0,07 mbar. Nach Kristallisation aus Diethylether/Diisopropylether erhält man das gereinigte Diastereomerengemisch in Form beiger Kristalle. Schmelzintervall 103-112 °C.

b) Herstellung des Endproduktes

In ein Gemisch von 250 ml 2n Natronlauge und 400 ml Diethylether werden unter starkem Rühren bei —5 °C 27,6 g 1-(2,4-Dichlorphenyl)-2-fluor-2-(1H-1,2,4-triazol-1-yl)ethanol eingetragen. Anschliessend lässt man bei —5 °C 20 ml Acetylchlorid zutropfen, rührt noch 1 Stunde bei der selben Temperatur und dann noch so lang bis das Reaktionsgemisch Raumtemperatur erreicht. Die Etherphase wird nun abgetrennt, mit Wasser neutral gewaschen, über Natriumsulfat getrocknet, filtriert und eingedampft. Man erhält 30 g eines hellen Oels, das durch Zugabe von Methanol kristallisiert. Nach Umkristallisation aus Diisopropylether/Cyclohexanon schmilzt das Produkt bei 105-106 °C.

Beispiel H4

Herstellung von

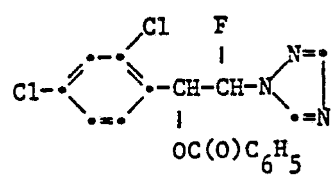

(1.15)

1-(2,4-Dichlorphenyl)-1-benzoyl-2-fluor-2-(1H-1,2,4-triazol-1-yl)-ethan

Zu einem Gemisch von 250 ml 2n Natronlauge und 27,6 g (nach Beispiel Hla hergestellten)1-(2,4-Dichlorphenyl)-2-fluor-2-(1H-1,2,4-triazol-1-yl)ethanol lässt man bei 0-5 °C unter kräftigem Rühren eine Lösung von 25 ml Benzoylchlorid in 250 ml Diethylether zutropfen. Das Reaktionsgemisch wird noch 2 Stunden bei 0-5 °C weitergerührt und dann ohne Kühlung bis es Raumtemperatur erreicht. Die abgetrennte Etherphase wird viermal mit je 50 ml 0,2n Natronlauge und anschliessend mit Wasser neutral gewaschen, über Natriumsulfat getrocknet, filtriert und eingedampft. Man erhält als Rohprodukt 40 g eines Oels, das bei 0,07 mbar und 180 °C durch Kurzwegdestillation gereinigt wird. Durch Umkristallisation aus Diisopropylether erhält man ca. 26 g des Produkts, das bei 104-108 °C schmilzt.

Beispiel H5

Herstellung von

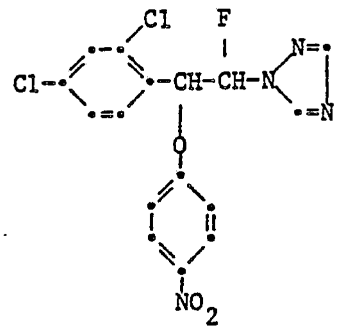

(2.1)

1-(2,4-Dichlorphenyl)-1-(4-nitrophenoxy)-2-fluor-2-(1H-1,2,4-triazol-1-yl)-ethan

In ein Gemisch von 250 ml 2n Natronlauge, 250 ml Diethylether und 0,5 g Tetrabutylammoniumbromid werden unter kräftigem Rühren 27,6 g 1-(2,4-Dichlorphenyl)-2-fluor-2-(1H-1,2,4-triazol-1-yl)ethanol (hergestellt nach Beispiel Hla) eingetragen. Zu dieser Mischung lässt man bei 20 °C 25 ml 4-Nitrofluorbenzol zutropfen und rührt noch 20 Stunden bei Raumtemperatur weiter. Die abgetrennte Etherphase wird mit Wasser neutral gewaschen, über Natriumsulfat getrocknet, filtriert und eingedampft. Das ölige Rohprodukt (35 g) wird säulenchromatographisch (Silikagel) gereinigt und schmilzt nach Umkristallisation am Diisopropylether/Cyclohexanon bei 98-100 °C.

Analog zu den beschriebenen Arbeitsweisen werden auch die nachfolgend aufgeführten Verbindungen der Formel I hergestellt :

(Siehe Tabellen Seite 17 ff.)

Tabelle 1 : Verbindungen der Formel

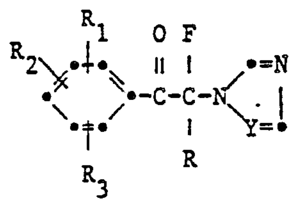

| Verb. Nr. | $R_1$ | $R_2$ | $R_3$ | R | Y | Physik. Konst. [°C] |
|---|---|---|---|---|---|---|
| 1.1 | 2-Cl | 4-Cl | H | H | N | Smp. 66-67 |
| 1.2 | 2-Cl | 4-Cl | H | H | CH | Sdp. 160/0,07 mbar |
| 1.3 | 2-Cl | 4-Cl | H | $CH_3$ | N | Smp. 64-65 |
| 1.4 | 2-Cl | 4-Cl | H | $CH_3$ | CH | Sdp. 160/0,13 mbar |
| 1.5 | 2-Cl | 4-Cl | H | $C_2H_5$ | N | Sdp. 150/0,07 mbar |
| 1.6 | 2-Cl | 4-Cl | H | $C_4H_9$-n | N | |
| 1.7 | 2-Cl | H | H | $C_6H_{13}$-n | N | |
| 1.8 | 2-Cl | H | H | H | N | Smp. 58-60 |
| 1.9 | H | H | H | H | N | Smp. 85-81 |
| 1.10 | H | H | H | $CH_3$ | N | Sdp.140/0,13 mbar |
| 1.11 | H | H | H | $C_4H_9$-n | N | Sdp.150/0,12 mbar |
| 1.12 | 2-Cl | 4-Cl | H | $C_3H_7$-n | N | Smp. 72-73 |
| 1.13 | 2-Cl | 4-Cl | H | $C_2H_5$ | CH | |
| 1.14 | H | 4-F | H | H | N | Smp. 93-95 |
| 1.15 | H | 4-F | H | $C_4H_9$-n | N | |
| 1.16 | H | 4-F | H | $CH_3$ | N | Oel, $n_D^{50}$ 1.544 |
| 1.17 | H | 4-F | H | $C_5H_{11}$-n | CH | |
| 1.18 | H | 4-Cl | H | H | N | Smp. 128-130 |
| 1.19 | H | 4-Cl | H | $CH_3$ | N | |

Tabelle 1 (Fortsetzung)

| Verb. Nr. | $R_1$ | $R_2$ | $R_3$ | R | Y | Physik. Konst. [°C] |
|---|---|---|---|---|---|---|
| 1.20 | H | 4-Cl | H | $C_2H_5$ | N | |
| 1.21 | H | 4-Cl | H | $C_2H_5$ | CH | |
| 1.22 | H | 4-Cl | H | $C_6H_{13}$-n | N | |
| 1.23 | 3-Cl | 4-Cl | H | H | N | Smp. 111-114 |
| 1.24 | 3-Cl | 4-Cl | H | $CH_3$ | N | |
| 1.25 | 2-Cl | H | 5-Cl | H | N | Smp. 65-66 |
| 1.26 | 3-Cl | 4-$CH_3$ | 5-Cl | H | N | |
| 1.27 | 2-$CH_3$ | 4-$CH_3$ | H | H | N | Smp. 92-95 |
| 1.28 | H | 4-Br | H | H | N | Smp. 150-152 |
| 1.29 | H | 4-Br | H | $CH_3$ | N | |
| 1.30 | H | 4-Br | H | $CH_3$ | CH | |
| 1.31 | H | 4-Br | H | $C_2H_5$ | N | |
| 1.32 | H | 4-Br | H | $C_4H_9$-n | CH | |
| 1.33 | H | 4-Br | H | $C_6H_{13}$-n | N | |
| 1.34 | 2-Cl | 4-F | H | H | N | $n_D^{50}$ 1.532 |
| 1.35 | 2-Cl | 4-F | H | $CH_3$ | N | |
| 1.36 | 2-Cl | 4-F | H | $C_2H_5$ | N | |
| 1.37 | H | 4-F | H | H | N | Smp. 65-66 |
| 1.38 | 2-Cl | 4-Br | H | H | N | |
| 1.39 | 2-Cl | 4-Br | H | $CH_3$ | N | |
| 1.40 | 2-Cl | 4-Br | H | $C_2H_5$ | N | |
| 1.41 | 2-Cl | 4-Br | H | $C_2H_5$ | CH | |

Tabelle 1 (Fortsetzung)

| Verb. Nr. | $R_1$ | $R_2$ | $R_3$ | R | Y | Physik. Konst. [°C] |
|---|---|---|---|---|---|---|
| 1.42 | 2-Cl | 4-Br | H | $C_4H_9$-n | N | |
| 1.43 | 2-Cl | 4-Br | H | $C_6H_{13}$-n | N | |
| 1.44 | 2-$CF_3$ | 4-Cl | H | H | N | |
| 1.45 | 2-$CF_3$ | 4-Cl | H | $CH_3$ | N | |
| 1.46 | 2-Cl | 4-$OCHF_2$ | H | H | N | |
| 1.47 | 2-Cl | 4-$OCHF_2$ | H | $CH_3$ | N | |
| 1.48 | 2-Cl | 4-$OCHF_2$ | H | $CH_3$ | CH | |
| 1.49 | 2-$OCHF_2$ | 4-Cl | H | H | N | |
| 1.50 | 2-$OCHF_2$ | 4-Cl | H | H | CH | |
| 1.51 | H | 4-CN | H | H | N | |
| 1.52 | 2-$OCH_3$ | 4-Cl | H | H | N | |
| 1.53 | 3-$OCH_3$ | 4-$OCH_3$ | 5-$OCH_3$ | H | N | |
| 1.54 | 2-$C_2H_5$ | 4-Cl | H | H | N | |
| 1.55 | 2-$CH_3$ | 4-Cl | H | H | N | |
| 1.56 | 2-F | 4-F | H | H | N | Smp. 53-55 |
| 1.57 | 2-Cl | 3-Cl | 4-Cl | H | N | Smp. 111-113 |
| 1.58 | H | 4-$OCH_3$ | H | H | N | Smp. 99-100 |
| 1.59 | H | 4-$OCH_3$ | H | $CH_3$ | N | Oel, $n_D^{50}$ 1.583 |
| 1.60 | H | 3-$OCH_3$ | H | H | N | Sdp. 150/0,07 mbar |
| 1.61 | H | 2-$OCH_3$ | H | H | N | Smp. 98-99 |

Tabelle 1 (Fortsetzung)

| Verb. Nr. | $R_1$ | $R_2$ | $R_3$ | R | Y | Physik. Konst. [°C] |
|---|---|---|---|---|---|---|
| 1.62 | 2-$CH_3$ | 4-$CH_3$ | H | $C_3H_7$-n | N | Sdp.150/0,1 mbar |
| 1.63 | 2-Cl | 3-Cl | H | H | N | Smp. 76-79 |
| 1.64 | 2-$C_2H_5$ | 4-$C_2H_5$ | H | H | N | Smp. 45-50 |
| 1.65 | 2-$CH_3$ | 4-$CH_3$ | 6-$CH_3$ | H | N | Smp. 86-88 |
| 1.66 | 2-$CH_3$ | 4-$CH_3$ | H | $CH_3$ | N | |
| 1.67 | 2-$CH_3$ | 4-$CH_3$ | H | $C_2H_5$ | N | |
| 1.68 | 2-$CH_3$ | 4-$CH_3$ | H | $C_3H_7$-n | N | |
| 1.69 | 2-$CH_3$ | 4-Cl | H | $CH_3$ | N | |
| 1.70 | 2-$CH_3$ | 4-Cl | H | $C_2H_5$ | N | |
| 1.71 | 2-$CH_3$ | 4-Cl | H | $C_3H_7$-n | N | |
| 1.72 | 2-Cl | 4-$CH_3$ | H | H | N | |
| 1.73 | 2-Cl | 4-$CH_3$ | H | $CH_3$ | N | |
| 1.74 | 2-Cl | 4-$CH_3$ | H | $C_2H_5$ | N | |
| 1.75 | 2-Cl | 4-$CH_3$ | H | $C_3H_7$-n | N | |
| 1.76 | 2-Cl | 4-$CF_3$ | H | H | N | |
| 1.77 | 2-Cl | 4-$CF_3$ | H | $CH_3$ | N | |
| 1.78 | 2-Cl | 4-$CF_3$ | H | $C_2H_5$ | N | |

Tabelle 2 : Verbindungen der Formel

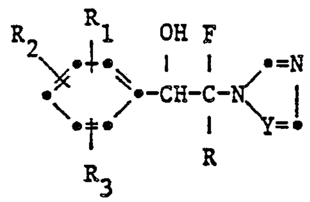

| Verb. Nr. | $R_1$ | $R_2$ | $R_3$ | R | Y | Physik. Konst. [°C] |
|---|---|---|---|---|---|---|
| 2.1 | 2-Cl | 4-Cl | H | H | N | Smp. 103-112 |
| 2.2 | 2-Cl | 4-Cl | H | H | CH | |
| 2.3 | 2-Cl | 4-Cl | H | $CH_3$ | N | Smp. 121-122 |
| 2.4 | 2-Cl | 4-Cl | H | $CH_3$ | CH | Smp. 109-113 |
| 2.5 | 2-Cl | 4-Cl | H | $C_2H_5$ | N | Smp. 113-122 |
| 2.6 | 2-Cl | 4-Cl | H | $C_4H_9$-n | N | |
| 2.7 | 2-Cl | 4-Cl | H | $C_6H_{13}$-n | N | |
| 2.8 | 2-Cl | H | H | H | N | Smp. 100-103 |
| 2.9 | H | H | H | H | N | Sdp. 150/0,07 mbar |
| 2.10 | H | H | H | $CH_3$ | N | Smp. 110-112 |
| 2.11 | H | H | H | $C_4H_9$-n | N | Smp. 117-120 |
| 2.12 | 2-Cl | 4-Cl | H | $C_3H_7$-n | N | Smp. 110-125 |
| 2.13 | 2-Cl | 4-Cl | H | $C_2H_5$ | CH | Smp. 187-194 |
| 2.14 | H | 4-F | H | H | N | Smp. 78-88 |
| 2.15 | H | 4-F | H | $C_4H_9$-n | CH | |
| 2.16 | H | 4-F | H | $CH_3$ | N | Smp. 146-148 |
| 2.17 | H | 4-Cl | H | H | N | Smp. 86-89 |

Tabelle 2 (Fortsetzung)

| Verb. Nr. | $R_1$ | $R_2$ | $R_3$ | R | Y | Physik. Konst. [°C] |
|---|---|---|---|---|---|---|
| 2.18 | H | 4-Cl | H | $CH_3$ | N | |
| 2.19 | H | 4-Cl | H | $C_2H_5$ | N | |
| 2.20 | H | 4-Cl | H | $C_2H_5$ | CH | |
| 2.21 | H | 4-Cl | H | $C_6H_{13}$-n | N | |
| 2.22 | 3-Cl | 4-Cl | H | H | N | Smp. 88-91 |
| 2.23 | 2-Cl | 3-Cl | H | $CH_3$ | N | |
| 2.24 | 2-Cl | H | 5-Cl | H | N | Smp. 153-155 |
| 2.25 | 3-Cl | 4-Cl | H | $CH_3$ | N | |
| 2.26 | 3-Cl | 4-$CH_3$ | 5-Cl | H | N | |
| 2.27 | 2-$CH_3$ | 4-$CH_3$ | H | H | N | Smp. 88-91 |
| 2.28 | H | 4-Br | H | H | N | Smp. 85-95 |
| 2.29 | 2-$CH_3$ | 4-$CH_3$ | H | $C_3H_7$-n | N | Smp. 81-95 |
| 2.30 | 2-Cl | 4-F | H | H | N | Smp. 29-31 |
| 2.31 | 2-Cl | 4-F | H | $CH_3$ | N | |
| 2.32 | 2-Cl | 4-F | H | $C_2H_5$ | N | |
| 2.33 | 2-Cl | 4-Br | H | H | N | |
| 2.34 | 2-Cl | 4-Br | H | $CH_3$ | N | |
| 2.35 | 2-$CF_3$ | 4-Cl | H | H | N | |
| 2.36 | 2-$CF_3$ | 4-Cl | H | $CH_3$ | N | |
| 2-37 | 2-Cl | 4-$OCHF_2$ | H | H | N | |
| 2.38 | 2-Cl | 4-$OCHF_2$ | H | $CH_3$ | N | |
| 2.39 | 2-Cl | 3-Cl | H | H | N | Smp. 122-126 |
| 2.40 | 2-$OCHF_2$ | 4-Cl | H | H | N | |

Tabelle 2 (Fortsetzung)

| Verb. Nr. | $R_1$ | $R_2$ | $R_3$ | R | Y | Physik. Konst. [°C] |
|---|---|---|---|---|---|---|
| 2.41 | 2-$C_2H_5$ | 4-$C_2H_5$ | H | H | N | Smp. 80-90 |
| 2.42 | 2-$CH_3$ | 4-$CH_3$ | 6-$CH_3$ | H | N | $n_D^{60}$ 1.540 |
| 2.43 | 2-$CH_3$ | 4-$CH_3$ | H | $CH_3$ | N | |
| 2.44 | 2-$CH_3$ | 4-$CH_3$ | H | $C_2H_5$ | N | |
| 2.45 | 2-$CH_3$ | 4-$CH_3$ | H | $C_3H_7$-n | N | |
| 2.46 | 2-$CH_3$ | 4-Cl | H | $CH_3$ | N | |
| 2.47 | 2-$CH_3$ | 4-Cl | H | $C_2H_5$ | N | |
| 2.48 | 2-$CH_3$ | 4-Cl | H | $C_3H_7$-n | N | |
| 2.49 | 2-Cl | 4-$CH_3$ | H | H | N | |
| 2.50 | H | 4-$NO_2$ | H | H | N | |
| 2.51 | H | 4-CN | H | H | N | |
| 2.52 | 2-$OCH_3$ | 4-Cl | H | H | N | |
| 2.53 | 3-$OCH_3$ | 4-$OCH_3$ | 5-$OCH_3$ | H | N | |
| 2.54 | 2-$C_2H_5$ | 4-$C_2H_5$ | H | H | N | Smp. 80-90 |
| 2.55 | 2-$CH_3$ | 4-$CH_3$ | 6-$CH_3$ | H | N | $n_D^{60}$ 1.540 |
| 2.56 | 2-F | 4-F | H | H | N | Smp. 132-134 |
| 2.57 | 2-Cl | 3-Cl | 4-Cl | H | N | Smp. 120-126 |
| 2.58 | H | 4-$OCH_3$ | H | H | N | Smp. 99-101 |
| 2.59 | H | 4-$OCH_3$ | H | $CH_3$ | N | Smp. 118-120 |
| 2.60 | H | 3-$OCH_3$ | H | H | N | Smp. 80-82 |
| 2.61 | H | 2-$OCH_3$ | H | H | N | Smp. 114-116 |
| 2.62 | 2-Cl | 4-$CH_3$ | H | $CH_3$ | N | |
| 2.63 | 2-Cl | 4-$CH_3$ | H | $C_2H_5$ | N | |
| 2.64 | 2-Cl | 4-$CH_3$ | H | $C_3H_7$-n | N | |
| 2.65 | 2-Cl | 4-$CF_3$ | H | H | N | |
| 2.66 | 2-Cl | 4-$CF_3$ | H | $CH_3$ | N | |
| 2.67 | 2-Cl | 4-$CF_3$ | H | $C_2H_5$ | N | |

Tabelle 5 : Ester der Formel

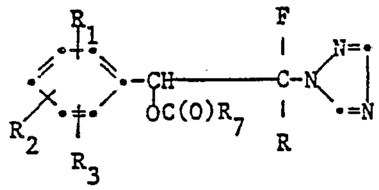

* Diastereomeres A
** Diastereomeres B
*** Diastereomerengemisch

| Verb. Nr. | $R_1$, $R_2$, $R_3$ | R | $R_7$ | physik. Konstante [°C] |
|---|---|---|---|---|
| 5.1 | 2,4-$Cl_2$ | H | $-CH_3$ | Smp. 105-106° |
| 5.2 | 2,4-$Cl_2$ | H | $-C_2H_5$ | |
| 5.3 | 2,4-$Cl_2$ | H | $-C_3H_7$(n) | Oel, $n_D^{50}$ 1.531 |
| 5.4 | 2,4-$Cl_2$ | H | $-C_3H_7$(i) | |
| 5.5 | 2,4-$Cl_2$ | H | $-C_4H_9$(n) | |
| 5.6 | 2,4-$Cl_2$ | H | $-CH_2OCH_3$ | |
| 5.7 | 2,4-$Cl_2$ | H | $-CH_2Cl$ | |
| 5.8 | 2,4-$Cl_2$ | H | $-CF_3$ | |
| 5.9 | 2,4-$Cl_2$ | H | -Cyclopropyl | Oel, $n_D^{50}$ 1.598 *** |
| 5.10 | 2,4-$Cl_2$ | H | -Cyclohexyl | Smp. 140-142° * |
| 5.11 | 2,4-$Cl_2$ | H | $-CH=CH-CH_3$ | Oel, $n_D^{50}$ 1.601 * |
| 5.12 | 2,4-$Cl_2$ | H | $-C\equiv CH$ | |
| 5.13 | 2,4-$Cl_2$ | H | -furyl(2) | Smp. ca. 30° *** |
| 5.14 | 2,4-$Cl_2$ | H | -tetrahydrofuryl(2) | |
| 5.15 | 2,4-$Cl_2$ | H | $-C_6H_5$ | Smp. 104-109° |
| 5.16 | 2,4-$Cl_2$ | H | $-C_6H_4Cl$(4) | Smp. 131-133° |
| 5.17 | 2,4-$Cl_2$ | H | $-C_6H_4Cl$(2) | Smp. 116-117° * |
| 5.18 | 2,4-$Cl_2$ | H | $-C_6H_3Cl_2$(2,4) | Smp. 85-87° |
| 5.19 | 2,4-$Cl_2$ | H | $-C_6H_3Cl_2$(2,6) | |
| 5.20 | 2,4-$Cl_2$ | H | $-C_6H_4CH_3$(4) | Smp. 155-156° |
| 5.21 | 2,4-$Cl_2$ | H | $-C_6H_4CF_3$(2) | |
| 5.22 | 2,4-$Cl_2$ | H | $-C_6H_4OCH_3$(4) | Smp. 137-139° * |
| 5.23 | 2,4-$Cl_2$ | H | $-C_6H_4OCHF_2$(4) | |

Tabelle 5 (Fortsetzung)

| Verb. Nr. | $R_1$, $R_2$, $R_3$ | R | $R_7$ | physik. Konstante [°C] |
|---|---|---|---|---|
| 5.24 | 2,4-$Cl_2$ | H | $-CH_2C_6H_5$ | Oel, $n_D^{50}$ 1.587 * |
| 5.25 | 2,4-$Cl_2$ | H | $-CH_2C_6H_4Cl(4)$ | |
| 5.26 | 2,4-$Cl_2$ | H | $-CH_2C_6H_4Cl(2)$ | |
| 5.27 | 2,4-$Cl_2$ | H | $-CH_2C_6H_4NO_2(4)$ | |
| 5.28 | 2,4-$Cl_2$ | $-CH_3$ | $-CH_3$ | |
| 5.29 | 2,4-$Cl_2$ | $-CH_3$ | $-C_2H_5$ | |
| 5.30 | 2,4-$Cl_2$ | $-CH_3$ | $-CH_2OCH_3$ | |
| 5.31 | 2,4-$Cl_2$ | $-CH_3$ | $-C_6H_5$ | |
| 5.32 | 2,4-$Cl_2$ | $-CH_3$ | -Furyl(2) | Oel, $n_D^{50}$ 1.731 |
| 5.33 | 2,4-$Cl_2$ | $-CH_3$ | -Tetrahydrofuryl(2) | |
| 5.34 | 2,4-$Cl_2$ | $-CH_3$ | $-C_6H_4Cl(4)$ | |
| 5.35 | 2,4-$Cl_2$ | $-C_2H_5$ | $-CH_3$ | |
| 5.36 | 2,4-$Cl_2$ | $-C_2H_5$ | $-C_2H_5$ | |
| 5.37 | 2,4-$Cl_2$ | $-C_2H_5$ | $-C_6H_{13}(n)$ | |
| 5.38 | 2,4-$Cl_2$ | $-C_2H_5$ | $-CH_2CH_2OCH_3$ | |
| 5.39 | 2,4-$Cl_2$ | $-C_2H_5$ | $-C_6H_5$ | Smp. 88-90° |
| 5.40 | 2-Cl, 4-F | -H | $-CH_3$ | |
| 5.41 | 2-Cl, 4-F | -H | $-C_2H_5$ | |
| 5.42 | 2-Cl, 4-F | -H | $-CH_2OCH_3$ | |
| 5.43 | 2-Cl, 4-F | -H | $-C_6H_5$ | |
| 5.44 | 2-Cl, 4-F | $-CH_3$ | $-CH_3$ | |
| 5.45 | 2-Cl, 4-F | $-CH_3$ | $-CH_2Cl$ | |
| 5.46 | 2-Cl, 4-F | $-CH_3$ | $-C_6H_5$ | |
| 5.47 | 2-Cl, 4-Br | -H | $-CH_3$ | |
| 5.48 | 2-Cl, 4-Br | -H | $-C_5H_{11}(n)$ | |
| 5.49 | 2-$CF_3$, 4-Cl | -H | $-CH_2OCH_3$ | |
| 5.50 | 2-$CF_3$, 4-Cl | -H | $-C_6H_5$ | |
| 5.51 | 2-$CF_3$, 4-Cl | $-CH_3$ | $-CH_3$ | |
| 5.52 | 2-$CF_3$, 4-Cl | $-CH_3$ | -Cyclopentyl | |
| 5.53 | 2-Cl, 4-$OCHF_2$ | -H | $-CH_3$ | |

Tabelle 5 (Fortsetzung)

| Verb. Nr. | $R_1$, $R_2$, $R_3$ | R | $R_7$ | physik. Konstante [°C] |
|---|---|---|---|---|
| 5.54 | 2-Cl, 4-$OCHF_2$ | H | $CH_2OCH_3$ | |
| 5.55 | 2-Cl, 4-$OCHF_2$ | H | $C_6H_5$ | |
| 5.56 | 2-Cl, 4-$OCHF_2$ | $CH_3$ | $C_2H_5$ | |
| 5.57 | 2-$CH_3$, 4-Cl | H | $C_6H_5$ | |
| 5.58 | 2-$CH_3$, 4-Cl | H | $CH_3$ | |
| 5.59 | 2,3-$Cl_2$ | H | $CH_3$ | |
| 5.60 | 2,3-$Cl_2$ | H | $CH_2OCH_3$ | |
| 5.61 | 2,4,6-$(CH_3)_3$ | H | $CH_3$ | |
| 5.62 | 2,4,6-$(CH_3)_3$ | H | $C_6H_5$ | Smp. 161-164° |
| 5.63 | 2,4-$Cl_2$ | H | $C_6H_3Cl_2$(3,4) | |
| 5.64 | 2,4-$Cl_2$ | H | Cyclopropyl | Smp. 115-117° ** |
| 5.65 | 2,4-$Cl_2$ | H | $C_6H_4Cl$(2) | Oel, $n_D^{50}$ 1.597 *** |
| 5.66 | 2,4-$Cl_2$ | H | 2-Furyl | Smp. 156-158° * |
| 5.67 | 2,4-$Cl_2$ | H | 2-Furyl | Smp. 101-103° ** |
| 5.68 | 2,4-$Cl_2$ | H | $C_6H_4OCH_3$(4) | Smp. 141-143° ** |
| 5.69 | 2,4-$Cl_2$ | H | $CH=CH-CH_3$ | Smp. 80-82° ** |
| 5.70 | 2,4-$Cl_2$ | H | $C_6H_4F$(4) | Smp. 130-132° * |
| 5.71 | 2,4-$Cl_2$ | H | $C_6H_4$(4) | Smp. 90-92° ** |
| 5.72 | 2,4-$Cl_2$ | H | Cyclohexyl | Oel, $n_D^{50}$ 1.630 ** |
| 5.73 | 2,4-$Cl_2$ | H | $C_6H_4CF_3$(4) | Smp. 118-119° * |
| 5.74 | 2,4-$Cl_2$ | H | $C_6H_4CF_3$(4) | Smp. 85-87° ** |
| 5.75 | 2,4-$Cl_2$ | H | $CH_2C_6H_5$ | Smp. 88-90° ** |
| 5.76 | 2,4-$Cl_2$ | $CH_3$ | $C_6H_3Cl_2$(2,4) | Oel, $n_D^{50}$ 1.888 |
| 5.77 | 2,4-$Cl_2$ | $CH_3$ | $C_6H_4F$(4) | Oel, $n_D^{50}$ 1.908 |
| 5.78 | 2,4-$Cl_2$ | $CH_3$ | $C_6H_4OCH_3$(4) | Oel, $n_D^{50}$ 1.899 ** |
| 5.79 | 2,4-$Cl_2$ | $CH_3$ | $C_6H_4OCH_3$(4) | Smp. 120-122° ** |
| 5.80 | 2,4-$Cl_2$ | H | $CH(C_2H_5)CH_2CH_3$ | Smp. 124-126° |
| 5.81 | 2,4-$Cl_2$ | H | $CH(C_2H_5)CH_2CH_3$ | Oel, $n_D^{50}$ 1.835 ** |
| 5.82 | 2,4-$Cl_2$ | $CH_3$ | $C_6H_4F$(4) | Oel, $n_D^{50}$ 1.794 |

Tabelle 5 (Fortsetzung)

| Verb. Nr. | $R_1$, $R_2$, $R_3$ | R | $R_7$ | physik. Konstante [°C] |
|---|---|---|---|---|
| 5.83 | 2,4-$Cl_2$ | H | $C_6H_4Cl(4)$ | Smp. 140-142° ** |
| 5.84 | 2,4-$Cl_2$ | $CH_3$ | $C_6H_4F(4)$ | Smp. 88-90° |
| 5.85 | 2,4-$Cl_2$ | $CH_3$ | $C_6H_3Cl_2(2,4)$ | Smp. 120-122° * |
| 5.86 | 2,4-$Cl_2$ | $CH_3$ | $C_6H_3Cl_2(2,4)$ | Smp. 102-104° ** |
| 5.87 | 2,4-$Cl_2$ | $C_2H_5$ | $C_6H_3Cl_2(2,4)$ | Oel, $n_D^{50}$ 1.877 |
| 5.88 | 2,4-$(CH_3)_2$ | H | $C_6H_5$ | |
| 5.89 | 2,4-$(CH_3)_2$ | H | $C_6H_4F(4)$ | |
| 5.90 | 2,4-$(CH_3)_2$ | H | $C_6H_4Cl(4)$ | |
| 5.91 | 2,4-$(CH_3)_2$ | H | $CH_3$ | |
| 5.92 | 2,4-$(CH_3)_2$ | H | $CH{=}CHCH_3$ | |
| 5.93 | 2,4-$(CH_3)_2$ | $CH_3$ | $C_6H_5$ | |
| 5.94 | 2,4-$(CH_3)_2$ | $CH_3$ | $C_6H_4CF_3(4)$ | |
| 5.95 | 2,4-$(CH_3)_2$ | $CH_3$ | 2-Furyl | |
| 5.96 | 2,4-$(CH_3)_2$ | $CH_3$ | $CH{=}CHCH_3$ | |
| 5.97 | 2,4-$(CH_3)_2$ | $C_2H_5$ | $CH_3$ | |
| 5.98 | 2,4-$(CH_3)_2$ | $C_2H_5$ | $C_6H_5$ | |
| 5.99 | 2,4-$(CH_3)_2$ | $C_2H_5$ | $C_6H_4F(4)$ | |
| 5.100 | 2,4-$(CH_3)_2$ | $C_3H_7$-n | $C_6H_5$ | |
| 5.101 | 2,4-$(CH_3)_2$ | $C_3H_7$-n | 2-Furyl | |

Tabelle 6 : Ether der Formel

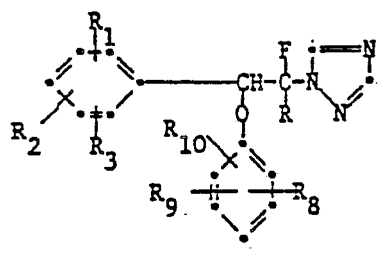

| Verb. Nr. | $R_1$, $R_2$, $R_3$ | R | $R_8$, $R_9$, $R_{10}$ | physik. Konstante [°C] |
|---|---|---|---|---|
| 6.1 | 2,4-$Cl_2$ | -H | 4-$NO_2$ | Smp. 98-100° |
| 6.2 | 2,4-$Cl_2$ | -H | H | |
| 6.3 | 2,4-$Cl_2$ | -H | 4-$NH_2$ | Smp. 142-144° |
| 6.4 | 2,4-$Cl_2$ | -H | 4-Cl | Sdp. 160°/0,05 mbar |
| 6.5 | 2,4-$Cl_2$ | -H | 4-$NHCOCH_3$ | Smp. 168-171° |
| 6.6 | 2,4-$Cl_2$ | -H | 3-$COOCH_3$, 4-$NO_2$ | |
| 6.7 | 2,4-$Cl_2$ | -H | 2-$NO_2$, 4-$COOCH_3$ | |
| 6.8 | 2,4-$Cl_2$ | -H | 2-$NO_2$, 4-$CF_3$ | |
| 6.9 | 2,4-$Cl_2$ | -H | 2-$NO_2$, 4-$OCH_3$ | Smp. 98-100° |
| 6.10 | 2,4-$Cl_2$ | -H | 2,6-$(NO_2)_2$, 4-CN | |
| 6.11 | 2,4-$Cl_2$ | -H | 2,6-$(NO_2)_2$, 4-$COOCH_3$ | |
| 6.12 | 2,4-$Cl_2$ | -H | 2,6-$(NO_2)_2$, 4-$CF_3$ | |
| 6.13 | 2,4-$Cl_2$ | -H | 2,4-$(NO_2)_2$ | |
| 6.14 | 2,4-$Cl_2$ | -H | 2,4-$(NO_2)_2$, 6-$CF_3$ | |
| 6.15 | 2,4-$Cl_2$ | -$CH_3$ | 4-$NO_2$ | |
| 6.16 | 2,4-$Cl_2$ | -$CH_3$ | 2,4-$(NO_2)_2$ | |
| 6.17 | 2,4-$Cl_2$ | -$CH_3$ | 2-$NO_2$, 4-$CF_3$ | |
| 6.18 | 2,4-$Cl_2$ | -$CH_3$ | 2,6-$(NO_2)_2$, 4-CN | |
| 6.19 | 2,4-$Cl_2$ | -$C_2H_5$ | 4-$NO_2$ | Harz, $n_D^{50}$ 1.599 |
| 6.20 | 2,4-$Cl_2$ | -$C_2H_5$ | 2,4-$(NO_2)_2$, 6-$CF_3$ | |
| 6.21 | 2,4-$Cl_2$ | -$C_2H_5$ | 2,4-$(NO_2)_2$ | |
| 6.22 | 4-Cl | -H | 4-$NO_2$ | |

Tabelle 6 (Fortsetzung)

| Verb. Nr. | $R_1$, $R_2$, $R_3$ | R | $R_8$, $R_9$, $R_{10}$ | physik. Konstante [°C] |
|---|---|---|---|---|
| 6.23 | 4-Cl | $-CH_3$ | 3-$COOCH_3$, 4-$NO_2$ | |
| 6.24 | 4-Cl | $-CH_3$ | 2-$NO_2$, 4-$CF_3$ | |
| 6.25 | 2-$CH_3$, 4-Cl | -H | 4-$NO_2$ | |
| 6.26 | 2-$CH_3$, 4-Cl | -H | 4-$NO_2$ | |
| 6.27 | 2-$CF_3$, 4-Cl | -H | 4-$NO_2$ | |
| 6.28 | 2-$CF_3$, 4-Cl | -H | 2,6-$(NO_2)_2$, 4-$COOCH_3$ | |
| 6.29 | 2-$CF_3$, 4-Cl | -H | 2-$NO_2$, 4-$CF_3$ | |
| 6.30 | 2-$CF_3$, 4-Cl | $-CH_3$ | 2-$NO_2$, 4-$CF_3$ | |
| 6.31 | 2-Cl, 4-Br | -H | 4-$NO_2$ | |
| 6.32 | 2-Cl, 4-Br | $-CH_3$ | 4-$NO_2$ | |
| 6.33 | 2-Cl, 4-$OCHF_2$ | -H | 4-$NO_2$ | |
| 6.34 | 2,4-$Cl_2$ | H | 4-$NO_2$ | Smp.102-105° * |
| 6.35 | 2,4-$Cl_2$ | H | 4-$NHCOCH_3$ | Smp.191-194° * |
| 6.36 | 2,4-$Cl_2$ | H | 4-$NH_2$ | Smp. 75-77° ** |
| 6.37 | 2,4-$(CH_3)_2$ | H | 4-$NO_2$ | |
| 6.38 | 2,4-$(CH_3)_2$ | H | 4-$NH_2$ | * |
| 6.39 | 2,4-$(CH_3)_2$ | H | 4-$NHCOCH_3$ | ** |
| 6.40 | 2,4-$(CH_3)_2$ | $CH_3$ | 4-$NO_2$ | |
| 6.41 | 2,4-$(CH_3)_2$ | $C_2H_5$ | 4-$NO_2$ | |
| 6.42 | 2,4-$(CH_3)_2$ | $C_3H_7$-n | 4-$NO_2$ | |

Formulierungsbeispiele für flüssige Wirkstoffe der Formel I (% = Gewichtsprozent)

| F1. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenyl-polyethylenglykol-ether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus den Tabellen | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| F3. Granulate | a) | b) |
|---|---|---|
| Wirkstoff aus den Tabellen | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

Formulierungsbeispiele für feste Wirkstoffe
der Formel I (% = Gewichtsprozent)

| F5. Spritzpulver | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F6. Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus den Tabellen | 5% | 8% |
| Talkum | 95% | - |
| Kaolin | - | 92% |

Man erhält anwendungsfertige Stäubemittel, indem der Wirkstoff mit den Träger vermischt auf einer geeigneten Mühle vermahlen wird.

F8. Extruder-Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

F9. Umhüllungs-Granulat

| | |
|---|---|
| Wirkstoff aus den Tabellen | 3 % |
| Polyethylenglykol (M G 200) | 3 % |
| Kaolin (MG = Molekulargewicht) | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Im nachfolgenden Beispiel B1 wurden die folgenden beiden Substanzen A und B des Standes der Technik vergleichend mitgeprüft :

(Siehe Schema Seite 32 f.)

| DE 2,431,407 | strukturell nächstvergleichbare Verbindungen der Formel I |
|---|---|
| 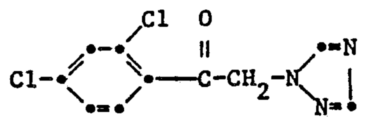 A (Verb. Nr. 12) | 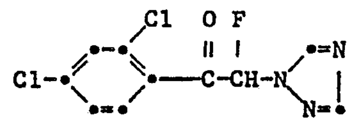 (Verb. Nr. 1.1) |
| 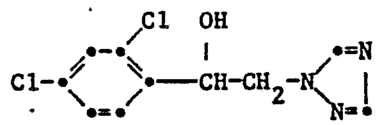 B (Verb. Nr. 5) | 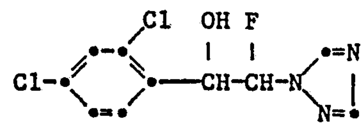 (Verb. Nr. 2.1) |

Beispiel B1 :
Wirkung gegen Piricularia oryzae auf Reis

a) Residual-protektive Wirkung

Reispflanzen wurden nach zweiwöchiger Anzucht mit einer aus Spritzpulver des Wirkstoffes hergestellten Spritzbrühe (0,02 % Aktivsubstanz) besprüht. Nach 48 Stunden wurden die behandelten Pflanzen mit einer Konidiensuspension des Pilzes infiziert. Nach 5 Tagen Inkubation bei 95-100 % relativer Luftfeuchtigkeit und 24 °C wurde der Pilzbefall beurteilt.

b) Systemische Wirkung

Zu zweiwöchigen Reispflanzen wurde eine aus Spritzpulver des Wirkstoffes hergestellte Spritzbrühe gegossen (0,006 % Aktivsubstanz bezogen auf das Erdvolumen). Darauf wurden die Töpfe mit Wasser soweit gefüllt, dass die untersten Stengelteile der Reispflanzen im Wasser standen. Nach 96 Stunden wurden die behandelten Reispflanzen mit einer Konidiensuspension des Pilzes infiziert. Nach einer Inkubation der infizierten Pflanzen während 5 Tagen bei 95-100 % relativer Luftfeuchtigkeit und ca. 24 °C wurde der Pilzbefall beurteilt.

Die unbehandelten aber infizierten Kontrollpflanzen zeigten in den beiden Tests a und b einen Pilzbefall von 100 %.

Reispflanzen, die mit Verbindungen aus den Tabellen 1 bis 6 behandelt worden waren, so z. B. mit einer der Verbindungen Nr. 1.1-1.5, 1.9-1.12, 1.14, 1.18, 1.23, 1.25, 1.28, 1.34, 1.37, 1.56-1.59, 1.62-1.65, 2.1, 2.3, 2.5, 2.9-2.14, 2.17, 2.22, 2.24, 2.28, 2.29, 2.30, 2.41, 2.42, 2.56, 5.1, 5.15-5.18, 5.20, 5.22, 5.32, 5.64-5.69, 5.70-5.78, 5.80-5.87, 6.1, 6.4, 6.5 und 6.34 zeigten eine Reduktion des Pilzbefalls auf 0 bis 5 %. Insbesondere die Verbindungen Nr. 1.1, 1.3, 1.4, 1.5, 2.1, 2.3, 2.4 und 2.5 unterdrücken den Pilzbefall sogar vollständig (0 % Befall). Demgegenüber bewirkten die Verbindungen A und B des Standes der Technik bei den geringen Testkonzentrationen keinerlei Hemmung des Piriculariabefalls und zeigten wie die unbehandelte Kontrolle einen Befall von 100 %.

Beispiel B2 :
Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,02 % Aktivsubstanz) besprüht. Nach 24 Stunden wurden die behandelten Pflanzen mit einer Uredosporensuspension des Pilzes infiziert. Nach einer Inkubation während 48

Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

b) Systemische Wirkung

Zu Weizenpflanzen wurden 5 Tage nach Aussaat eine aus Spritzpulver des Wirkstoffes hergestellte Spritzbrühe gegossen (0,006 % Aktivsubstanz bezogen auf das Bodenvolumen). Nach 72 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen aus den Tabellen zeigten gegen Puccinia-Pilze eine sehr gute Wirkung. Unbehandelte aber infizierte Kontrollpflanzen zeigten einen Puccinia-Befall von 100 %. Unter anderen hemmten die Verbindungen Nr. 1.1-1.5, 1.8-1.10, 1.12, 1.14, 1.18, 1.23, 1.28, 1.34, 1.56, 1.58, 1.61, 1.64, 1.65, 2.1-2.5, 2.8-2.14, 2.16, 2.17, 2.24, 2.28-2.30, 2.39, 2.41, 2.42, 2.54, 2.56, 2.58, 5.1, 5.3, 5.13, 5.15, 5.20, 5.65-5.76, 5.80-5.86, 6.5 und 6.9 den Puccinia-Befalls fast vollständig (0 bis 5 % Befall).

Beispiel B3 :
Wirkung gegen Erysiphae graminis auf Gerste

a) Residual-protektive Wirkung

Ca. 8 cm hohe Gerstenpflanzen wurden mit einer aus Spritzpulver des Wirkstoffes hergestellten Spritzbrühe (0,02 % Aktivsubstanz) besprüht. Nach 24 Stunden wurden die behandelten Pflanzen mit Konidien des Pilzes bestäubt. Die infizierten Gerstenpflanzen wurden in einem Gewächshaus bei ca 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 72 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen der Formel I zeigten gute Wirkung gegen Erysiphe-Pilze. Unbehandelte, aber infizierte Kontrollpflanzen zeigten einen Erysiphe-Befall von 100 %. Unter anderen Verbindungen aus der Tabelle hemmten die Verbindungen Nr. 1.1-1.5, 1.8-1.12, 1.14, 1.16, 1.18, 1.23, 1.25, 1.27, 1.34, 1.37, 1.56-1.65, 2.1, 2.3-2.5, 2.8-2.14, 2.17, 2.27-2.30, 2.39, 2.41, 2.42, 2.54, 2.59, 2.61, 5.1, 5.3, 5.9, 5.11, 5.13, 5.15, 5.17, 5.18, 5.20, 5.22, 5.62, 5.64-5.87, 6.1, 6.3, 6.5, 6.19, 6.34 und 6.35 den Pilzbefall bei Gerste auf 0 bis 5 %, insbesondere die Verbindungen Nr. 1.1 und 2.1 bewirken eine vollständige (0 % Befall).

**Patentansprüche**

1. Verbindungen der Formel I'

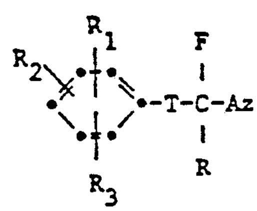

(I')

worin

Az für 1H-1,2,4-Triazol, 4H-1,2,4-Triazol oder 1H-Imidazol steht ;

T für —C(O)—, —CH(OH)— oder für eine der Gruppen

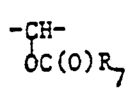 oder 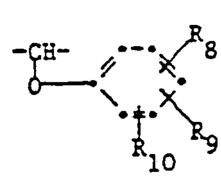 steht, wobei

$R_7$ unsubstituiertes oder durch $C_1$-$C_3$-Alkoxy, Halogen oder Cyano substituiertes $C_1$-$C_6$-Alkyl, $C_3$-$C_6$-Alkenyl, $C_3$-$C_6$-Alkinyl, $C_3$-$C_6$-Cycloalkyl, 2-Furyl, 2-Tetrahydrofuryl oder unsubstituiertes oder durch Halogen, Nitro, $C_1$-$C_3$-Alkyl, $C_1$-$C_3$-Alkoxy, $C_1$-$C_3$-Haloalkyl und/oder $C_1$-$C_3$-Haloalkoxy substituiertes Phenyl oder Benzyl bedeutet ;

$R_8$, $R_9$ und $R_{10}$ unabhängig voneinander für Wasserstoff, Nitro, Halogen, Cyano, $C_1$-$C_3$-Alkyl, $C_1$-$C_3$-Alkoxy, $C_1$-$C_3$-Haloalkoxy, —COO($C_1$-$C_3$-Alkyl), $NH_2$ oder $NHCOCH_3$ stehen ;

R Wasserstoff oder $C_1$-$C_6$-Alkyl bedeutet ;

$R_1$ und $R_2$ unabhängig voneinander für Wasserstoff, Halogen, $C_1$-$C_6$-Alkyl, $C_1$-$C_6$-Alkoxy, $C_1$-$C_6$-Haloalkyl, $C_1$-$C_6$-Haloalkoxy, Nitro und/oder Cyano stehen ; und

$R_3$ Wasserstoff, Halogen, $C_1$-$C_6$-Alkyl, $C_1$-$C_6$-Alkoxy, $C_1$-$C_6$-Haloalkyl, $C_1$-$C_6$-Haloalkoxy, Nitro und/oder Cyano repräsentiert ;

unter Einschluss ihrer Säureadditionssalze und Metallsalzkomplexe ; mit der Massgabe, dass 1) wenn T gleich —C(O)— ist, R $C_1$-$C_6$-Alkyl darstellt und 2) wenn T gleich C(O)— ist und gleichzeitig von den Substituenten $R_1$, $R_2$ und $R_3$ zwei Reste 2,4-Difluor und der dritte Rest Wasserstoff bedeuten, R $C_2$-$C_6$-Alkyl darstellt.

2. Verbindungen der Formel I' nach Anspruch 1, worin

Az für 1H-1,2,4-Triazol oder 1H-Imidazol steht ;

T für —C(O)— oder —CH(OH)— steht ;

R Wasserstoff oder $C_1$-$C_6$-Alkyl bedeutet ;

$R_1$ und $R_2$ unabhängig voneinander Wasserstoff, Halogen, $C_1$-$C_3$-Alkyl, $C_1$-$C_3$-Alkoxy, $C_1$-$C_3$-Haloalkyl, $C_1$-$C_3$-Haloalkoxy, Nitro und/oder Cyano stehen ; und

$R_3$ Wasserstoff, Halogen, $C_1$-$C_3$-Alkyl, $C_1$-$C_3$-Alkoxy, $C_1$-$C_3$-Haloalkyl, $C_1$-$C_3$-Haloalkoxy, Nitro und/oder Cyano repräsentiert ;

unter Einschluss ihrer Säureadditionssalze und Metallkomplexe.

3. Verbindungen der Formel I' nach Anspruch 1, worin

Az für 1H-1,2,4-Triazol oder 1H-Imidazol steht ;

T für eine der Gruppen

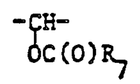 oder 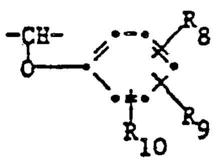 steht, wobei

$R_7$ $C_1$-$C_6$-Alkyl oder durch $C_1$-$C_2$-Alkoxy, Fluor, Chlor oder Brom substituiertes $C_1$-$C_4$-Alkyl, $C_3$-$C_4$-Alkenyl, $C_3$-$C_4$-Alkinyl, Cyclopropyl, Cyclohexyl, 2-Furyl, 2-Tetrahydrofuryl oder unsubstituiertes oder durch Fluor, Chlor, Brom, Methyl, Methoxy, Nitro, $CF_3$, $OCF_2H$, und/oder $OCF_3$ substituiertes Phenyl oder Benzyl bedeutet ;

$R_8$, $R_9$ und $R_{10}$ unabhängig voneinander für Wasserstoff, Nitro, Fluor, Chlor, Brom, Cyano, Methyl, Methoxy, —COO($CH_3$), $NH_2$ oder $NHCOCH_3$ stehen ;

R Wasserstoff oder $C_1$-$C_6$-Alkyl bedeutet ;

$R_1$ und $R_2$ unabhängig voneinander für Wasserstoff, Halogen, $C_1$-$C_3$-Alkyl, $C_1$-$C_3$-Alkoxy, $C_1$-$C_3$-Haloalkyl, $C_1$-$C_3$-Haloalkoxy, Nitro und/oder Cyano stehen ; und

$R_3$ Wasserstoff, Halogen, $C_1$-$C_6$-Alkyl, $C_1$-$C_3$-Alkoxy, $C_1$-$C_3$-Haloalkyl, $C_1$-$C_3$-Haloalkoxy, Nitro und/oder Cyano repräsentiert ;

unter Einschluss ihrer Säureadditionssalze und Metallkomplexe.

4. Verbindungen der Formel I' nach Anspruch 2, worin T für —C(O)— oder —CH(OH)— steht ; Az für 1H-1,2,4-Triazol steht ; R für Wasserstoff oder $C_1$-$C_4$-Alkyl steht ; $R_1$ in ortho-Position und $R_2$ in para-Position stehen und unabhängig voneinander für Fluor, Chlor, Brom, Methyl, Methoxy, $CF_3$, $OCHF_2$, $OCF_3$, Nitro oder Cyano bedeuten ; und $R_3$ für Wasserstoff steht ; unter Einschluss ihrer Säureadditionssalze und Metallkomplexe.

5. Eine Verbindung der Formel I' nach Anspruch 2, ausgewählt aus der Reihe :

1-(2,4-Dichlorphenyl)-2-fluor-2-(1H-1,2,4-triazol-1-yl)-propanon (Nr. 1.3) ;

1-(2,4-Dichlorphenyl)-2-fluor-2-(1H-imidazol-1-yl)-propanon (Nr. 1.4) ;

1-(2,4-Dichlorphenyl)-2-fluor-2-(1H-1,2,4-triazol-1-yl)-butanon (Nr. 1.5) ;

1-(Phenyl)-2-fluor-2-(1H-1,2,4-triazol-1-yl)-propanon (Nr. 1.10) ;

1-(Phenyl)-2-fluor-2-(1H-1,2,4-triazol-1-yl)-hexanon (Nr. 1.11) ;

1-(2,4-Dichlorphenyl)-2-fluor-2-(1H-1,2,4-triazol-1-yl)-pentanon (Nr. 1.12) ;

1-(4-Fluorphenyl)-2-fluor-2-(1H-1,2,4-triazol-1-yl)-hexanon (Nr. 1.15) ;

6. Eine Verbindung der Formel I' nach Anspruch 2, ausgewählt aus der Reihe :

1-(2,4-Dichlorphenyl)-2-fluor-2-(1H-1,2,4-triazol-1-yl)-ethanol (Nr. 2.1) ;

1-(2,4-Dichlorphenyl)-2-fluor-2-(1H-1,2,4-triazol-1-yl)-propanol (Nr. 2.3) ;

1-(2,4-Dichlorphenyl)-2-fluor-2-(1H-imidazol-1-yl)-propanol (Nr. 2.4) ;

1-(2,4-Dichlorphenyl)-2-fluor-2-(1H-1,2,4-triazol-1-yl)-butanol (Nr. 2.5) ;

1-(Phenyl)-2-fluor-2-(1H-1,2,4-triazol-1-yl)-ethanol (Nr. 2.9) ;

1-(Phenyl)-2-fluor-2-(1H-1,2,4-triazol-1-yl)-propanol (Nr. 2.10) ;
1-(Phenyl)-2-fluor-2-(1H-1,2,4-triazol-1-yl)-hexanol (Nr. 2.11) ;
1-(2,4-Dichlorphenyl)-2-fluor-2-(1H-1,2,4-triazol-1-yl)-pentanol (Nr. 2.12) ;
1-(2,4-Dichlorphenyl)-2-fluor-2-(1H-imidazol-1-yl)-butanol (Nr ; 2.13) ;
1-(4-Fluorphenyl)-2-fluor-2-(1H-1,2,4-triazol-1-yl)-ethanol (Nr. 2.14) ;
1-(4-Chlorphenyl)-2-fluor-2-(1H-1,2,4-triazol-1-yl-ethanol (Nr. 2.17) ;
1-(3,4-Dichlorphenyl)-2-fluor-2-(1H-1,2,4-triazol-1-yl-ethanol (Nr. 2.22) ;
1-(2,5-Dichlorphenyl)-2-fluor-2-(1H-1,2,4-triazol-1-yl-ethanol (Nr. 2.24) ;
1-(2,4-Dimethylphenyl)-2-fluor-2-(1H-1,2,4-triazol-1-yl-ethanol (Nr. 2.27) ;
1-(4-Bromphenyl)-2-fluor-2-(1H-1,2,4-triazol-1-yl-ethanol (Nr. 2.28) ;
1-(2,4-Difluorphenyl)-2-fluor-2-(1H-1,2,4-triazol-1-yl-ethanol (Nr. 2.56) ;
1-(2,3,4-Trichlorphenyl)-2-fluor-2-(1H-1,2,4-triazol-1-yl-ethanol (Nr. 2.57) ;
1-(3-Methoxyphenyl)-2-fluor-2-(1H-1,2,4-triazol-1-yl)-ethanol (Nr. 2.60) ;
1-(2-Methoxyphenyl)-2-fluor-2-(1H-1,2,4-triazol-1-yl)-ethanol (Nr. 2.61) ;

7. Eine Verbindung der Formel I' nach Anspruch 3, ausgewählt aus der Reihe :
1-(2,4-Dichlorphenyl)-1-acetoyl-2-fluor-2-(1H-1,2,4-triazol-1-yl)-ethan,
1-(2,4-Dichlorphenyl)-1-benzoyl-2-fluor-2-(1H-1,2,4-triazol-1-yl-ethan,
1-(2,4-Dichlorphenyl)-1-(4-nitrophenoxy)-2-fluor-2-(1H-1,2,4-triazol-1-yl)-ethan.

8. Verfahren zur Herstellung von Verbindungen der Formel I' gemäss Anspruch 1, dadurch gekennzeichnet, dass man ein Keton der Formel II

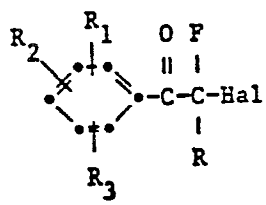
(II)

mit einem Azol der Formel III

Me—Az (III)

zu einer Verbindung der Formel Ia

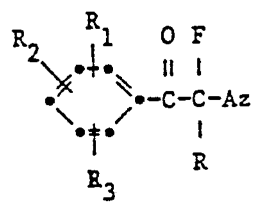
(Ia)

reagieren lässt und letztere gegebenenfalls zu einem Alkohol der Formel Ib

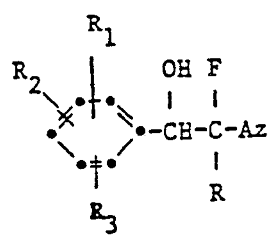
(Ib)

reduziert und an die Verbindungen der Formel Ia und Ib, sofern gewünscht, eine Säure oder eine Metallsalz addiert, oder das Alkanol der Formel Ib durch Reaktion mit einer Verbindung der Formel IV

$R_7$—C(O)—Y (IV)

verestert oder zur Herstellung der Ether der Formel I', indem man obigen Alkohol der Formel Ib durch Reaktion mit einer Verbindung der Formel V

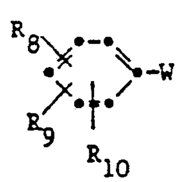
(V)

verethert, und gegebenenfalls eine verfahrensgemäss erhältliche Verbindung durch Reduktion, Umwandlung oder Austausch eines Substituenten in eine andere Verbindung der Formel I überführt, wobei die Substituenten $R_1$, $R_2$, $R_3$, $R_7$, $R_8$, $R_9$, $R_{10}$, R und Az wie unter Formel I' definiert sind, Y für Hydroxy, Alkoxy, $R_7C(O)O$— oder Halogen steht und W für OH oder eine Abgangsgruppe steht.

9. Mittel zur Bekämpfung oder Verhütung eines Befalls durch Mikroorganismen, dadurch gekennzeichnet, dass es als mindestens eine aktive Komponente eine Verbindung der Formel I

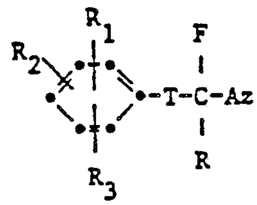 (I)

worin

Az für 1H-1,2,4-Triazol, 4H-1,2,4-Triazol oder 1H-Imidazol steht ;

T für —C(O)—, —CH(OH)— oder für eine der Gruppen

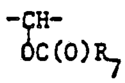 oder 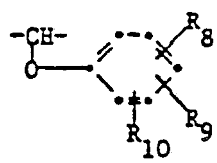 steht, wobei

$R_7$ unsubstituiertes oder durch $C_1$-$C_3$-Alkoxy, Halogen oder Cyano substituiertes $C_1$-$C_6$-Alkyl, $C_3$-$C_6$-Alkenyl, $C_3$-$C_6$-Alkinyl, $C_3$-$C_6$-Cycloalkyl, 2-Furyl, 2-Tetrahydrofuryl oder unsubstituiertes oder durch Halogen, Nitro, $C_1$-$C_3$-Alkyl, $C_1$-$C_3$-Alkoxy, $C_1$-$C_3$-Haloalkyl und/oder $C_1$-$C_3$-Haloalkoxy substituiertes Phenyl oder Benzyl bedeutet ;

$R_8$, $R_9$ und $R_{10}$ unabhängig voneinander für Wasserstoff, Nitro, Halogen, Cyano, $C_1$-$C_3$-Alkyl, $C_1$-$C_3$-Alkoxy, $C_1$-$C_3$-Haloalkoxy, —COO($C_1$-$C_3$-Alkyl), $NH_2$ oder $NHCOCH_3$ stehen ;

R Wasserstoff oder $C_1$-$C_6$-Alkyl bedeutet ;

$R_1$ und $R_2$ unabhängig voneinander für Wasserstoff, Halogen, $C_1$-$C_6$-Alkyl, $C_1$-$C_6$-Alkoxy, $C_1$-$C_6$-Haloalky, $C_1$-$C_6$-Haloalkoxy, Nitro und/oder Cyano stehen ;

$R_3$ Wasserstoff, Halogen, $C_1$-$C_6$-Alkyl, $C_1$-$C_6$-Alkoxy, $C_1$-$C_6$-Haloalkyl, $C_1$-$C_6$-Haloalkoxy, Nitro und/oder Cyano repräsentiert ; unter Einschluss ihrer Säureadditionssalze und Metallsalzkomplexe.

10. Verfahren zur Bekämpfung oder Verhütung eines Befalls von Kulturpflanzen durch phytopathogene Mikroorganismen, dadurch gekennzeichnet, dass man eine Verbindung der Formel I

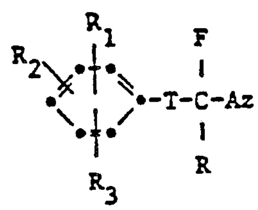 (I)

worin

Az für 1H-1,2,4-Triazol, 4H-1,2,4-Triazol oder 1H-Imidazol steht ;

T für —C(O)—, —CH(OH)— oder für eine der Gruppen

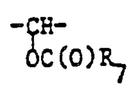 oder 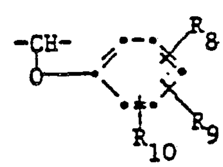 steht, wobei

$R_7$ unsubstituiertes oder durch $C_1$-$C_3$-Alkoxy, Halogen oder Cyano substituiertes $C_1$-$C_6$-Alkyl, $C_3$-$C_6$-Alkenyl, $C_3$-$C_6$-Alkinyl, $C_3$-$C_6$-Cycloalkyl, 2-Furyl, 2-Tetrahydrofuryl oder unsubstituiertes oder durch Halogen, Nitro, $C_1$-$C_3$-Alkyl, $C_1$-$C_3$-Alkoxy, $C_1$-$C_3$-Haloalkyl und/oder $C_1$-$C_3$-Haloalkoxy substituiertes Phenyl oder Benzyl bedeutet ;

$R_8$, $R_9$ und $R_{10}$ unabhängig voneinander für Wasserstoff, Nitro, Halogen, Cyano, $C_1$-$C_3$-Alkyl, $C_1$-$C_3$-Alkoxy, $C_1$-$C_3$-Haloalkoxy, —COO($C_1$-$C_3$-Alkyl), $NH_2$ oder $NHCOCH_3$ stehen ;

R Wasserstoff oder $C_1$-$C_6$-Alkyl bedeutet ;

$R_1$ und $R_2$ unabhängig voneinander für Wasserstoff, Halogen $C_1$-$C_6$-Alkyl, $C_1$-$C_6$-Alkoxy, $C_1$-$C_6$-Haloalkyl, $C_1$-$C_6$-Haloalkoxy, Nitro und/oder Cyano stehen ;

$R_3$ Wasserstoff, Halogen, $C_1$-$C_6$-Alkyl, $C_1$-$C_6$-Alkoxy, $C_1$-$C_6$-Haloalkyl, $C_1$-$C_6$-Haloalkoxy, Nitro und/oder Cyano repräsentiert ; unter Einschluss ihrer Säureadditionssalze und Metallkomplexe.

**Claims**

1. Compounds of formula I'

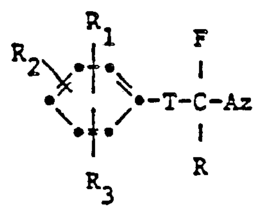

(I')

wherein

Az is 1H-1,2,4-triazole, 4H-1,2,4-triazole or 1H-imidazole ;

T is —C(O)—, —CH(OH)— or one of the groups

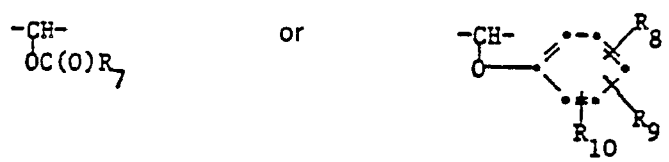

wherein

$R_7$ is $C_1$-$C_6$alkyl which is unsubstituted or substituted by $C_1$-$C_3$alkoxy, halogen or cyano, or is $C_3$-$C_6$alkenyl, $C_3$-$C_6$alkynyl, $C_3$-$C_6$cycloalkyl, 2-furyl, 2-tetrahydrofuryl, or is phenyl or benzyl, each unsubstituted or substituted by halogen, nitro $C_1$-$C_3$alkyl, $C_1$-$C_3$alkoxy, $C_1$-$C_3$-haloalkyl and/or $C_1$-$C_3$haloalkoxy ;

$R_8$, $R_9$ and $R_{10}$ are each independently hydrogen, nitro, halogen, cyano, $C_1$-$C_3$alkyl, $C_1$-$C_3$alkoxy $C_1$-$C_3$haloalkoxy, —COO($C_1$-$C_3$alkyl), $NH_2$ or $NHCOCH_3$ ;

R is hydrogen or $C_1$-$C_6$alkyl ;

$R_1$ and $R_2$ are each independently hydrogen, halogen, $C_1$-$C_6$alkyl, $C_1$-$C_6$alkoxy, $C_1$-$C_6$-haloalkyl, $C_1$-$C_6$-haloalkoxy, nitro and/or cyano ; and

$R_3$ is hydrogen, halogen, $C_1$-$C_6$alkyl, $C_1$-$C_6$alkoxy, $C_1$-$C_6$haloalkyl, $C_1$-$C_6$haloalkoxy, nitro and/or cyano ;

or an acid addition salt or metal complex thereof ; with the proviso that 1) when T is —C(O)—, R is $C_1$-$C_6$alkyl and 2) when T is C(O)— and, at the same time, of the substituents $R_1$, $R_2$ and $R_3$ two radicals are difluoro and the third radical is hydrogen, R is $C_2$-$C_6$alkyl.

2. Compounds of formula I' according to claim 1, wherein

Az is 1H-1,2,4-triazole or 1H-imidazole ;

T is —C(O)— or —CH(OH)— ;

R is hydrogen or $C_1$-$C_6$alkyl ;

$R_1$ and $R_2$ are each independently hydrogen, halogen, $C_1$-$C_3$alkyl, $C_1$-$C_3$alkoxy, $C_1$-$C_3$-haloalkyl, $C_1$-$C_3$haloalkoxy, nitro and/or cyano ; and

$R_3$ is hydrogen, halogen, $C_1$-$C_3$alkyl, $C_1$-$C_3$alkoxy, $C_1$-$C_3$haloalkyl, $C_1$-$C_3$haloalkoxy, nitro and/or cyano ;

or an acid addition salt or metal complex thereof.

3. Compounds of formula I' according to claim 1, wherein

Az is 1H-1,2,4-triazole or 1H-imidazole ;

T is one of the groups

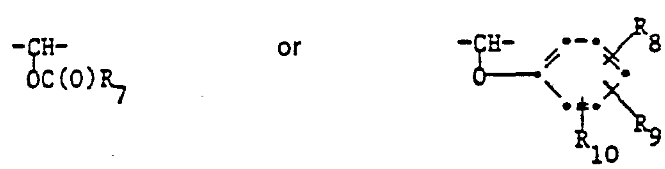

wherein

$R_7$ is $C_1$-$C_6$alkyl, or $C_1$-$C_4$alkyl substituted by $C_1$-$C_2$alkoxy, fluorine, chlorine or bromine, or is $C_3$-

$C_4$alkenyl, $C_3$-$C_4$alkynyl, cyclopropyl, cyclohexyl, 2-furyl, 2-tetrahydrofuryl, or is phenyl or benzyl, each unsubstituted or substituted by fluorine, chlorine, bromine, methyl, methoxy, nitro, $CF_3$, $OCF_2H$ and/or $OCF_3$ ;

$R_8$, $R_9$ and $R_{10}$ are each independently hydrogen, nitro, fluorine, chlorine, bromine, cyano, methyl, methoxy, —$COO(CH_3)$, $NH_2$ or $NHCOCH_3$ ;

R is hydrogen or $C_1$-$C_6$alkyl ;

$R_1$ and $R_2$ are each independently hydrogen, halogen, $C_1$-$C_3$alkyl, $C_1$-$C_3$alkoxy, $C_1$-$C_3$haloalkyl, $C_1$-$C_3$haloalkoxy, nitro and/or cyano ; and

$R_3$ is hydrogen, halogen, $C_1$-$C_6$alkyl, $C_1$-$C_3$alkoxy, $C_1$-$C_3$haloalkyl, $C_1$-$C_3$haloalkoxy, nitro and/or cyano ;

or an acid addition salt or metal complex thereof.

4. Compounds of formula I' according to claim 2, wherein T is —C(O)— or —CH(OH)— ; Az is 1H-1,2,4-triazole ; R is hydrogen or $C_1$-$C_4$alkyl ; $R_1$ is in ortho-position and $R_2$ is in para-position and each is independently fluorine, chlorine, bromine, methyl, methoxy, $CF_3$, $OCHF_2$, $OCF_3$, nitro or cyano ; and $R_3$ is hydrogen ; or an acid addition salt or metal complex thereof.

5. A compound of formula I' according to claim 2 selected from the group consisting of
1-(2,4-dichlorophenyl)-2-fluoro-2-(1H-1,2,4-triazol-1-yl)propanone (1.3) ;
1-(2,4-dichlorophenyl)-2-fluoro-2-(1H-imidazol-1-yl)-propanone (1.4) ;
1-(2,4-dichlorophenyl)-2-fluoro-2-(1H-1,2,4-triazol-1-yl)butanone (1.5) ;
1-(phenyl)-2-fluoro-2-(1H-1,2,4-triazol-1-yl)propanone (1.10) ;
1-(phenyl)-2-fluoro-2-(1H-1,2,4-triazol-1-yl)hexanone (1.11) ;
1-(2,4-dichlorophenyl)-2-fluoro-2-(1H-1,2,4-triazol-1-yl)pentanone (1.12) ;
1-(4-fluorophenyl)-2-fluoro-2-(1H-1,2,4-triazol-1-yl)-hexanone (1.15).

6. A compound of formula I' according to claim 2 selected from the group consisting of
1-(2,4-dichlorophenyl)-2-fluoro-2-(1H-1,2,4-triazol-1-yl) ethanol (2.1) ;
1-(2,4-dichlorophenyl)-2-fluoro-2-(1H-1,2,4-triazol-1-yl)propanol (2.3) ;
1-(2,4-dichlorophenyl)-2-fluoro-2-(1H-imidazol-1-yl)propanol (2.4) ;
1-(2,4-dichlorophenyl)-2-fluoro-2-(1H-1,2,4-triazol-1-yl)butanol (2.5) ;
1-(phenyl)-2-fluoro-2-(1H-1,2,4-triazol-1-yl)ethanol (2.9) ;
1-(phenyl)-2-fluoro-2-(1H-1,2,4-triazol-1-yl)propanol (2.10) ;
1-(phenyl)-2-fluoro-2-(1H-1,2,4-triazol-1-yl)hexanol (2.11) ;
1-(2,4-dichlorophenyl)-2-fluoro-2-(1H-1,2,4-triazol-1-yl)pentanol (2.12) ;
1-(2,4-dichlorophenyl)-2-fluoro-2-(1H-imidazol-1-yl)-butanol (2.13) ;
1-(4-fluorophenyl)-2-fluoro-2-(1H-1,2,4-triazol-1-yl)-ethanol (2.14) ;
1-(4-chlorophenyl)-2-fluoro-2-(1H-1,2,4-triazol-1-yl)-ethanol (2.17) ;
1-(3,4-dichlorophenyl)-2-fluoro-2-(1H-1,2,4-triazol-1-yl)ethanol (2.22) ;
1-(2,5-dichlorophenyl)-2-fluoro-2-(1H-1,2,4-triazol-1-yl)ethanol (2.24) ;
1-(2,4-dimethylphenyl)-2-fluoro-2-(1H-1,2,4-triazol-1-yl)ethanol (2.27) ;
1-(4-bromophenyl)-2-fluoro-2-(1H-1,2,4-triazol-1-yl)-ethanol (2.28) ;
1-(2,4-difluorophenyl)-2-fluoro-2-(1H-1,2,4-triazol-1-yl)ethanol (2.56) ;
1-(2,3,4-trichlorophenyl)-2-fluoro-2-(1H-1,2,4-triazol-1-yl)ethanol (2.57) ;
1-(3-methoxyphenyl)-2-fluoro-2-(1H-1,2,4-triazol-1-yl)ethanol (2.60) ;
1-(2-methoxyphenyl)-2-fluoro-2-(1H-1,2,4-triazol-1-yl)ethanol (2.61).

7. A compound of formula I' according to claim 3 selected from the group consisting of
1-(2,4-dichlorophenyl)-1-acetoyl-2-fluoro-2-(1H-1,2,4-triazol-1-yl)ethane,
1-(2,4-dichlorophenyl)-1-benzoyl-2-fluoro-2-(1H-1,2,4-triazol-1-yl)ethane,
1-(2,4-dichlorophenyl)-1-(4-nitrophenoxy)-2-fluoro-2-(1H-1,2,4-triazol-1-yl)ethane.

8. A process for the preparation of compounds of formula I' according to claim 1, characterised in that a ketone of formula II

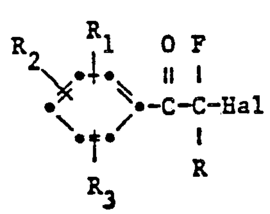

(II)

is reacted with an azole of formula III

$$Me—Az \quad (III)$$

to give a compound of formula Ia

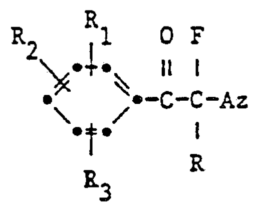

(Ia)

and optionally the latter is reduced to give an alcohol of formula Ib

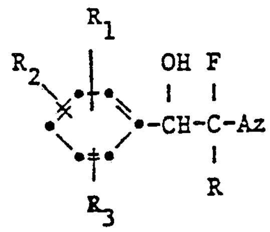

(Ib)

and, if desired, an acid or a metal salt is added to the compounds of the formula Ia and Ib, or the alkanol of formula Ib is esterified by reaction with a compound of formula IV

$$R_7—C(O)—Y \quad (IV)$$

or the alcohol of formula Ib above is etherified by reaction with a compound of formula V

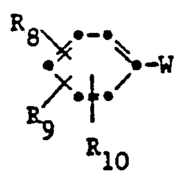

(V)

to give an ether of formula I', and optionally a compound obtainable by a process of this invention is converted into another compound of formula I by reduction, conversion or by exchange or a substituent, in which formulae II, III, Ia, Ib, IV and V above $R_1$, $R_2$, $R_3$, $R_7$, $R_8$, $R_9$, $R_{10}$, R and Az are as defined for formula I', Y is hydroxy, alkoxy, $R_7C(O)O—$ or halogen, and W is OH or a leaving group.

9. A composition for controlling microorganisms or preventing attack by such microorganisms, characterised in that the composition contains, as at least one active ingredient, a compound of formula I

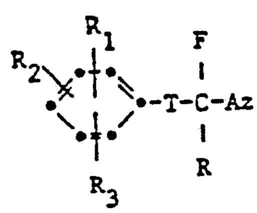

(I)

wherein

Az is 1H-1,2,4-triazole, 4H-1,2,4-triazole or 1H-imidazole ;

T is —C(O)—, —CH(OH)— or one of the groups

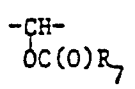

or

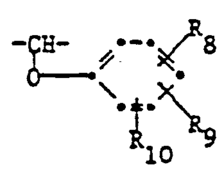

wherein

$R_7$ is $C_1$-$C_6$alkyl which is unsubstituted or substituted by $C_1$-$C_3$alkoxy, halogen or cyano, or is $C_3$-$C_6$-alkenyl, $C_3$-$C_6$alkynyl, $C_3$-$C_6$cycloalkyl, 2-furyl, 2-tetrahydrofuryl, or is phenyl or benzyl, each unsubstituted or substituted by halogen, nitro, $C_1$-$C_3$alkyl, $C_1$-$C_3$alkoxy, $C_1$-$C_3$haloalkyl and/or $C_1$-$C_3$haloalkoxy ;

$R_8$, $R_9$ and $R_{10}$ are each independently hydrogen, nitro, halogen, cyano, $C_1$-$C_3$-alkyl, $C_1$-$C_3$alkoxy, $C_1$-$C_3$haloalkoxy, —COO($C_1$-$C_3$alkyl), $NH_2$ or $NHCOCH_3$ ;

R is hydrogen or $C_1$-$C_6$alkyl ;

$R_1$ and $R_2$ are each independently hydrogen, halogen, $C_1$-$C_6$alkyl, $C_1$-$C_6$alkoxy, $C_1$-$C_6$haloalkyl, $C_1$-$C_6$haloalkoxy, nitro and/or cyano ; and

$R_3$ is hydrogen, halogen, $C_1$-$C_6$alkyl, $C_1$-$C_6$alkoxy, $C_1$-$C_6$haloalkyl, $C_1$-$C_6$haloalkoxy, nitro and/or cyano ;

or an acid addition salt or metal complex thereof.

10. A method of controlling phytopathogenic microorganisms or of protecting cultivated plants from attack by such microorganisms, characterised in that a compound of formula I

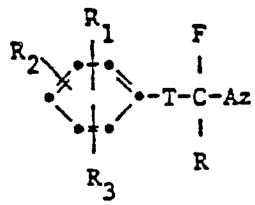

(I)

wherein

Az is 1H-1,2,4-triazole, 4H-1,2,4-triazole or 1H-imidazole ;

T is —C(O)—, —CH(OH)— or one of the groups.

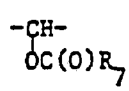

or

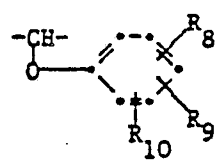

wherein

$R_7$ is $C_1$-$C_6$alkyl which is unsubstituted or substituted by $C_1$-$C_3$alkoxy, halogen or cyano, or is $C_3$-$C_6$-alkenyl, $C_3$-$C_6$alkynyl, $C_3$-$C_6$cycloalkyl, 2-furyl, 2-tetrahydrofuryl, or is phenyl or benzyl, each unsubstituted or substituted by halogen, nitro, $C_1$-$C_3$alkyl, $C_1$-$C_3$alkoxy, $C_1$-$C_3$haloalkoxy and/or $C_1$-$C_3$haloalkoxy ;

$R_8$, $R_9$ and $R_{10}$ are each independently hydrogen, nitro, halogen, cyano, $C_1$-$C_3$alkyl, $C_1$-$C_3$alkoxy, $C_1$-$C_3$haloalkoxy, —COO($C_1$-$C_3$alkyl), $NH_2$ or $NHCOCH_3$ ;

R is hydrogen or $C_1$-$C_6$alkyl ;

$R_1$ and $R_2$ are each independently hydrogen, halogen, $C_1$-$C_6$alkyl, $C_1$-$C_6$alkoxy, $C_1$-$C_6$haloalkyl, $C_1$-$C_6$haloalkoxy, nitro and/or cyano ; and

$R_3$ is hydrogen, halogen, $C_1$-$C_6$alkyl, $C_1$-$C_6$alkoxy, $C_1$-$C_6$haloalkyl, $C_1$-$C_6$haloalkoxy, nitro and/or cyano ;

or an acid addition salt or metal complex thereof.

## Revendications

1. Composés de formule I'

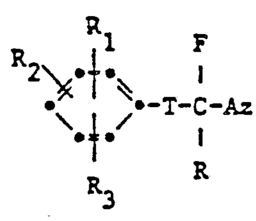

(I')

dans laquelle

Az représente un cycle 1H-1,2,4-triazole, 4H-1,2,4-triazole ou 1H-imidazole ;

T représente —C(O)—, —CH(OH)— ou l'un des groupes

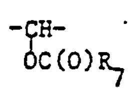

ou

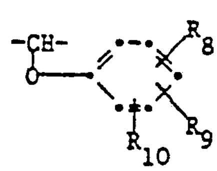

dans lesquels

$R_7$ représente un groupe alkyle en C1-C6, alcényle en C3-C6, alcynyle en C3-C6, cycloalkyle en C3-C6, 2-furyle, 2-tétrahydrofuryle non substitué ou substitué par des groupes alcoxy en C1-C3, des halogènes ou des groupes cyano, ou un groupe phényle ou benzyle non substitué ou substitué par des halogènes, des groupes nitro, alkyle en C1-C3, alcoxy en C1-C3, halogénoalkyle en C1-C3 et/ou halogénoalcoxy en C1-C3 ;

$R_8$, $R_9$ et $R_{10}$ représentent chacun, indépendamment les uns des autres, l'hydrogène, un groupe nitro, un halogène, un groupe cyano, alkyle en C1-C3, alcoxy en C1-C3, halogénoalcoxy en C1-C3, —COO(alkyle en C1-C3), $NH_2$ ou $NHCOCH_3$ ;

R représente l'hydrogène ou un groupe alkyle en C1-C6 ;

$R_1$ et $R_2$ représentent chacun, indépendamment l'un de l'autre, l'hydrogène, un halogène, un groupe alkyle en C1-C6, alcoxy en C1-C6, halogénoalkyle en C1-C6, halogénoalcoxy en C1-C6, nitro et/ou cyano ; et

$R_3$ représente l'hydrogène, un halogène, un groupe alkyle en C1-C6, alcoxy en C1-C6, halogénoalkyle en C1-C6, halogénoalcoxy en C1-C6, nitro et/ou cyano ;

y compris leurs sels formés par addition avec des acides et complexes de sels métalliques ;
sous réserve que 1) lorsque T représente —C(O)—, R représente un groupe alkyle en C1-C6, et 2) lorsque T représente —C(O)— et que, simultanément, parmi les symboles $R_1$, $R_2$ et $R_3$, deux de ces symboles représentent des substituants difluoro et le troisième l'hydrogène, R représente un groupe alkyle en C2-C6.

2. Composés de formule I' selon la revendication 1, dans lesquels

Az représente un cycle 1H-1,2,4-triazole ou 1H-imidazole ;

T représente —C(O)— ou —CH(OH)— ;

R représente l'hydrogène ou un groupe alkyle en C1-C6 ;

$R_1$ et $R_2$ représentent chacun, indépendamment l'un de l'autre, l'hydrogène, un halogène, un groupe alkyle en C1-C3, alcoxy en C1-C3, halogénoalkyle en C1-C3, halogénoalcoxy en C1-C3, nitro et/ou cyano ; et

$R_3$ représente l'hydrogène, un halogène, un groupe alkyle en C1-C3, alcoxy en C1-C3, halogénoalkyle en C1-C3, halogénoalcoxy en C1-C3, nitro et/ou cyano ;

y compris leurs sels formés par addition avec des acides et complexes métalliques.

3. Composés de formule I' selon la revendication I, dans lesquels

Az représente un cycle 1H-1,2,4-triazole ou 1H-imidazole ;

T représente l'un des groupes

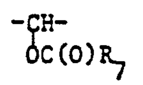

ou

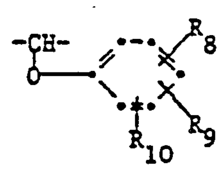

dans lesquels

$R_7$ représente un groupe alkyle en C1-C6 ou un groupe alkyle en C1-C4, alcényle en C3-C4, alcynyle en C3-C4, cyclopropyle, cyclohexyle, 2-furyle, 2-tétrahydrofuryle substitué par des groupes alcoxy en C1-C2, le fluor, le chlore ou le brome, ou un groupe phényle ou benzyle non substitué ou substitué par le fluor, le chlore, le brome, des groupes méthyle, méthoxy, nitro, $CF_3$, $OCF_2H$ et/ou $OCF_3$ ;

$R_8$, $R_9$ et $R_{10}$ représentent chacun, indépendamment les uns des autres, l'hydrogène, un groupe nitro, le fluor, le chlore, le brome, un groupe cyano, méthyle, méthoxy, $—COO(CH_3)$, $NH_2$ ou $NHCOCH_3$ ;

R représente l'hydrogène ou un groupe alkyle en C1-C6 ;

$R_1$ et $R_2$ représentent chacun, indépendamment l'un de l'autre, l'hydrogène, un halogène, un groupe alkyle en C1-C3, alcoxy en C1-C3, halogénoalkyle en C1-C3, halogénoalcoxy en C1-C3, nitro et/ou cyano ; et

$R_3$ représente l'hydrogène, un halogène, un groupe alkyle en C1-C6, alcoxy en C1-C3, halogénoalkyle en C1-C3, halogénoalcoxy en C1-C3, nitro et/ou cyano ;

y compris leurs sels formés par addition avec des acides et complexes métalliques.

4. Composés de formule I' selon la revendication 2, dans lesquels T représente —C(O)— ou —CH(OH)— ; Az représente un cycle 1H-1,2,4-triazole ; R représente l'hydrogène ou un groupe alkyle en C1-C4 ; $R_1$ est en position ortho et $R_2$ en position para et ils représentent chacun, indépendamment l'un de l'autre, le fluor, le chlore, le brome, un groupe méthyle, méthoxy, $CF_3$, $OCHF_2$, $OCF_3$, nitro ou cyano ; et $R_3$ représente l'hydrogène ; y compris leurs seuls formés par addition avec des acides et complexes métalliques.

5. Un composé de formule I' selon la revendication 2, choisi dans le groupe suivant :

1-(2,4-dichlorophényl)-2-fluoro-2-(1H-1,2,4-triazole-1-yl)-propanone (n° 1.3) ;

1-(2,4-dichlorophényl)-2-fluoro-2-(1H-imidazole-1-yl)-propanone (n° 1.4) ;

1-(2,4-dichlorophényl)-2-fluoro-2-(1H-1,2,4-triazole-1-yl)-butanone (n° 1.5) ;

1-(phényl)-2-fluoro-2-(1H-1,2,4-triazole-1-yl)-propanone (n° 1.10) ;

1-(phényl)-2-fluoro-2-(1H-1,2,4-triazole-1-yl)-hexanone (n° 1.11) ;
1-(2,4-dichlorophényl)-2-fluoro-2-(1H-1,2,4-triazole-1-yl)-pentanone (n° 1.12) ;
1-(4-fluorophényl)-2-fluoro-2-(1H-1,2,4-triazole-1-yl)-hexanone (n° 1.15).

6. Un composé de formule I' selon la revendication 2, choisi dans le groupe suivant :
1-(2,4-dichlorophényl)-2-fluoro-2-(1H-1,2,4-triazole-1-yl)-éthanol (n° 2.1) ;
1-(2,4-dichlorophényl)-2-fluoro-2-(1H-1,2,4-triazole-1-yl)-propanol (n° 2.3) ;
1-(2,4-dichlorophényl)-2-fluoro-2-(1H-imidazole-1-yl)-propanol (n° 2.4) ;
1-(2,4-dichlorophényl)-2-fluoro-2-(1H-1,2,4-triazole-1-yl)-butanol (n° 2.5) ;
1-(phényl)-2-fluoro-2-(1H-1,2,4-triazole-1-yl)-éthanol (n° 2.9) ;
1-(phényl)-2-fluoro-2-(1H-1,2,4-triazole-1-yl)-propanol (n° 2.10) ;
1-(phényl)-2-fluoro-2-(1H-1,2,4-triazole-1-yl)-hexanol (n° 2.11) ;
1-(2,4-dichlorophényl)-2-fluoro-2-(1H-1,2,4-triazole-1-yl)-pentanol (n° 2.12) ;
1-(2,4-dichlorophényl)-2-fluoro-2-(1H-imidazole-1-yl)-butanol (n° 2.13) ;
1-(4-fluorophényl)-2-fluoro-2-(1H-1,2,4-triazole-1-yl)-éthanol (n° 2.14) ;
1-(4-chlorophényl)-2-fluoro-2-(1H-1,2,4-triazole-1-yl)-éthanol (n° 2.17) ;
1-(3,4-dichlorophényl)-2-fluoro-2-(1H-1,2,4-triazole-1-yl)-éthanol (n° 2.22) ;
1-(2,5-dichlorophényl)-2-fluoro-2-(1H-1,2,4-triazole-1-yl)-éthanol (n° 2.24) ;
1-(2,4-diméthylphényl)-2-fluoro-2-(1H-1,2,4-triazole-1-yl)-éthanol (n° 2.27) ;
1-(4-bromophényl)-2-fluoro-2-(1H-1,2,4-triazole-1-yl)-éthanol (n° 2.28) ;
1-(2,4-difluorophényl)-2-fluoro-2-(1H-1,2,4-triazole-1-yl)-éthanol (n° 2.56) ;
1-(2,3,4-trichlorophényl)-2-fluoro-2-(1H-1,2,4-triazole-1-yl)-éthanol (n° 2.57) ;
1-(3-méthoxyphényl)-2-fluoro-2-(1H-1,2,4-triazole-1-yl)-éthanol (n° 2.60) ;
1-(2-méthoxyphényl)-2-fluoro-2-(1H-1,2,4-triazole-1-yl)-éthanol (n° 2.61).

7. Un composé de formule I' selon la revendication 3 choisi dans le groupe suivant :
1-(2,4-dichlorophényl)-1-acétoyl-2-fluoro-2-(1H-1,2,4-triazole-1-yl)-éthane ;
1-(2,4-dichlorophényl)-1-benzoyl-2-fluoro-2-(1H-1,2,4-triazole-1-yl)-éthane ;
1-(2,4-dichlorophényl)-1-(4-nitrophénoxy)-2-fluoro-2-(1H-1,2,4-triazole-1-yl)-éthane.

8. Procédé de préparation des composés de formule I' selon la revendication 1, caractérisé en ce que l'on fait réagir une cétone de formule II

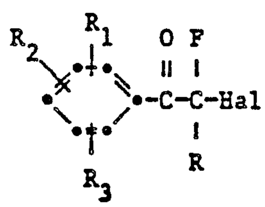

(II)

avec un azole de formule III

Me—Az (III)

ce qui donne un composé de formule Ia

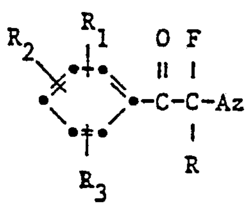

(Ia)

qu'on réduit le cas échéant en un alcool de formule Ib

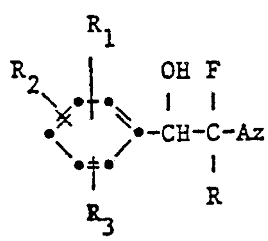

(Ib)

après quoi, si on le désire, on fixe un acide ou un sel métallique par addition sur les composés de

formules Ia et Ib, ou bien on estérifie l'alcanol de formule Ib par réaction avec un composé de formule IV

$R_7$—C(O)—Y (IV)

ou bien, lorsqu'on veut préparer les éthers de formule I', on éthérifie l'alcool de formule Ib ci-dessus par réaction avec un composé de formule V

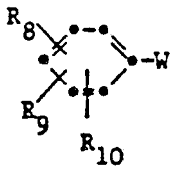

(V)

et, le cas échéant, on convertit un composé ainsi obtenu, par réduction, transposition ou échange d'un substituant contre un autre, en une autre composé de formule I, les symboles $R_1$, $R_2$, $R_3$, $R_7$, $R_8$, $R_9$, $R_{10}$, R et Az ayant les significations indiquées en référence à la formule I', Y représente un groupe hydroxy, alcoxy, $R_7C(O)O$— ou un halogène et W représente un groupe OH ou un groupe éliminable.

9. Produit pour prévenir ou combattre une infestation par des microorganismes, caractérisé en ce qu'il contient au moins un composant actif consistant en un composé de formule I

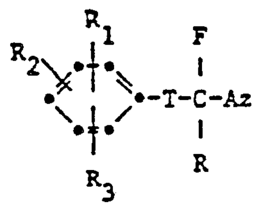

(I)

dans laquelle

Az représente un cycle 1H-1,2,4-triazole, 4H-1,2,4-triazole ou 1H-imidazole ;

T représente —C(O)—, CH(OH)— ou l'un des groupes

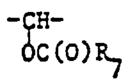

ou

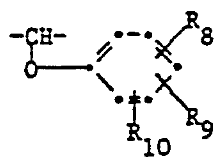

dans lesquels

$R_7$ représente un groupe alkyle en C1-C7, alcényle en C3-C6, alcynyle en C3-C6, cycloalkyle en C3-C6, 2-furyle, 2-tétrahydrofuryle non substitué ou substitué par des groupes alcoxy en C1-C3, des halogènes ou des groupes cyano, ou un groupe phényle ou benzyle non substitué ou substitué par des halogènes, des groupes nitro, alkyle en C1-C3, alcoxy en C1-C3, halogénoalkyle en C1-C3 ou halogénoalcoxy en C1-C3 ;

$R_8$, $R_9$ et $R_{10}$ représentent chacun, indépendamment les uns des autres, l'hydrogène, un groupe nitro, un halogène, un groupe cyano, alkyle en C1-C3, alcoxy en C1-C3, halogénoalcoxy en C1-C3, —COO(alkyle en C1-C3), $NH_2$ ou $NHCOCH_3$ ;

R représente l'hydrogène ou un groupe alkyle en C1-C6 ;

$R_1$ et $R_2$ représentent chacun, indépendamment l'un de l'autre, l'hydrogène, un halogène, un groupe alkyle en C1-C6, alcoxy en C1-C6, halogénoalkyle en C1-C6, halogénoalcoxy en C1-C6, nitro et/ou cyano ;

$R_3$ représente l'hydrogène, un halogène, un groupe alkyle en C1-C6, alcoxy en C1-C6, halogénoalkyle en C1-C6, halogénoalcoxy en C1-C6, nitro et/ou cyano ;

y compris leurs sels formés par addition avec des acides et complexes de sels métalliques.

10. Procédé pour prévenir ou combattre une infestation de végétaux cultivés par des microorganismes phytopathogènes, caractérisé en ce que l'on utilise un composé de formule I

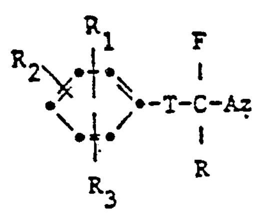

(I)

dans laquelle

Az représente un cycle 1H-1,2,4-triazole, 4H-1,2,4-triazole oi 1H-imidazole,

T représente —C(O)—, —CH(OH)— ou l'un des groupes

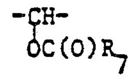

ou

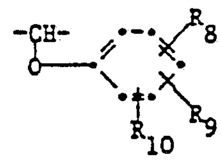

dans lesquels

$R_7$ représente un groupe alkyle en C1-C6, alcényle en C3-C6, alcynyle en C3-C6, cycloalkyle en C3-C6, 2-furyle, 2-tétrahydrofuryle non substitué ou substitué par des groupes alcoxy en C1-C3, des halogènes ou des groupes cyano, ou un groupe phényle ou benzyle non substitué ou substitué par des halogènes, des groupes nitro, alkyle en C1-C3, alcoxy en C1-C3, halogénoalkyle en C1-C3 et/ou halogénoalcoxy en C1-C3 ;

$R_8$, $R_9$ et $R_{10}$ représentent chacun, indépendamment les uns des autres, l'hydrogène, un groupe nitro, un halogène, un groupe cyano, alkyle en C1-C3, alcoxy en C1-C3, halogénoalcoxy en C1-C3, —COO(alkyle en C1-C3), $NH_2$ ou $NHCOCH_3$ ;

R représente l'hydrogène ou un groupe alkyle en C1-C6 ;

$R_1$ et $R_2$ représentent chacun, indépendamment l'un de l'autre, l'hydrogène, un halogène, un groupe alkyle en C1-C6, alcoxy en C1-C6, halogénoalkyle en C1-C6, halogénoalcoxy en C1-C6, nitro et/ou cyano ;

$R_3$ représente l'hydrogène, un halogène, un groupe alkyle en C1-C6, alcoxy en C1-C6, halogénoalkyle en C1-C5, halogénoalcoxy en C1-C6, nitro et/ou cyano ;

y compris ses sels formés par addition avec des acides et complexes métalliques.